# EUROPEAN PATENT SPECIFICATION

(11) **EP 3 802 677 B1**
(45) Date of publication and mention of the grant of the patent: **17.04.2024**
(21) Application number: 19717986.4
(22) Date of filing: 08.04.2019
(51) Int. Cl.: C08J 9/28, C08G 65/32, A61K 9/14, A61K 47/69

(54) **MESOPOROUS ELASTOMER**
MESOPORÖSES ELASTOMER
ÉLASTOMÈRE MÉSOPOREUX

(30) Priority: 25.05.2018 US 201862676391 P
(43) Date of publication of application: 14.04.2021
(73) Proprietor: Amferia AB, 43183 Mölndal (SE)
(72) Inventor: RAJASEKHARAN, Anand Kumar, 412 60 GÖTEBORG (SE); ANDERSSON, Martin, 431 38 MÖLNDAL (SE)
(74) Representative: Henricson Briggs, David James
(86) International application number: PCT/SE2019/050320
(87) International publication number: WO 2019/226086

(56) References cited:
- WO-A1-01/04234
- WO-A1-2017/156299
- CN-A- 107 245 136
- US-A1- 2006 194 927
- US-B2- 7 090 788
- DI BIASE M. ET AL: "Photopolymerisation of Pluronic diacrylate : a colloid-templated polymerisation", SOFT MATTER, vol. 7, 2011, pages 4928-4936, XP002792643,

## Description

### TECHNICAL FIELD

The invention relates a multifunctional and mechanically tough elastomeric material with improved handling and with an amphiphilic, ordered and mesoporous structure, and to the production thereof.

### BACKGROUND

Elastomers, such as natural rubber or silicone, are an important class of materials that possess high elasticity, flexibility and resilience. This renders them valuable in broad areas of applications, such as durable automobile parts, rubber sealants, flexible electronics, etc. Furthermore, owing to their impressive mechanical properties, elastomers have become extremely important in healthcare, especially in flexible and tough biomedical devices such as joint implants, facial prosthesis, breast implants, catheters and skin grafts.

Elastomers are composed of polymeric chains that are loosely held together by a combination of physical and chemical crosslinks, which helps them deform elastically, typically up to 500-1000% elongation, and recover instantaneously under dynamic loads. Over the past decade, a library of synthetic elastomers has been developed with a range of stiffness, maximum elongation and toughness, specifically determined by the type of polymeric molecule and the nature of the molecular network. Prominent examples are elastomers with reversible linkages, double networks, sacrificial bonds and nanoparticle reinforcement.

Synthetic elastomers produced today are randomly crosslinked polymer networks at the molecular level, which makes them homogenous, unstructured and isotropic at the mesoscale. Research in the field of bioinspired materials have shown that mesoscale architectural features (mesostructure), such as an ordered nanostructure with monodisperse mesoporosity and mesoscale anisotropy, are important for the functional properties of many applications including for biomaterials. For example, Therefore, it is desirable to design tough and elastic elastomers with well-defined mesostructural features.

The biggest challenge in designing nano-micro (meso) structured, 3D bulk elastomers is finding a good balance between obtaining the desired mesostructure in a macroscopic 3D material while (1) maintaining mechanical and biocompatible properties relevant to the current synthetic elastomers and (2) efficient processing methods to transform them into three-dimensional bulk materials. Promising developments have arisen in the field of synthetic hydrogels, where soft materials have been formed with highly-ordered nano-micro structures with ordered nanoporosity. However, hydrogels are ≈60-80% water, which makes them rapidly degradable and mechanically weak, leading to poor handling, which is nowhere comparable to synthetic elastomers in terms of properties, such as tensile and compressive strength and elasticity.

Another important aspect for elastomers is injectability. Elastomers that can be locally injected, for example in a medical setting, can be beneficial for applications, such as joint fillers or for soft-tissue applications like muscle grafts. This offers the possibility for minimally invasive surgery and efficient recovery. Today, injectable medical grade materials are usually in the form of (a) hydrogels, which are mechanically weak and degradable, and (b) poly(methyl methacrylate)-based polymers, which are often hard and brittle and does not display elastic or flexible behavior. Thus, the combined properties of a controlled mesostructure, high toughness and elasticity, biocompatibility together with efficient processability at room temperature, would enable the application of new elastomers in various areas including biomedical fields.

US 7,090,788 B2 (TDA Research, Inc.) relates to a nanoporous composite formed by an lyotropic liquid crystal (LLC) and a hydrophobic polymer in the hydrophobic regions of the LLC. The LLC is formed by polymerized and crosslinked amphiphilic monomers, and subsequent to the formation of the LLC an optionally crosslinked hydrophobic polymer may be added to form the composite.

Thus, there is a need for mechanically tough and elastic elastomers that are singular materials at the molecular and nanostructural level, with improved handling; and that can be efficiently processed into macroscopic objects.

### SUMMARY

Accordingly, the present invention preferably seeks to mitigate, alleviate or eliminate one or more of the above-identified deficiencies in the art singly or in any combination and solves at least the above mentioned problems by providing a mesoporous elastomer having an ordered and periodic nanoporous structure. The mesoporous elastomer is a solid, dry material which displays highly elastic deformation and has improved handling characteristics.

According to another aspect a precursor of such a mesoporous elastomer, i.e., an elastomeric lyotropic liquid crystal is provided.

Methods for the production of mesoporous elastomers and elastomeric lyotropic liquid crystals are also provided.

Further advantageous features of the invention are defined in the dependent claims. In addition, advantageous features of the invention are elaborated in embodiments disclosed herein.

### BRIEF DESCRIPTION OF THE DRAWINGS

The embodiments, together with further objects and advantages thereof, may best be understood by making reference to the following description taken together with the accompanying drawings, in which:

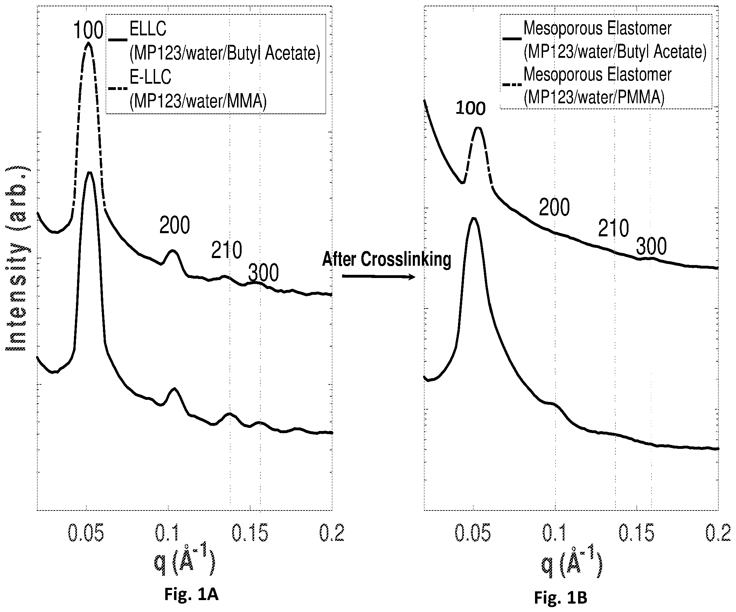

Fig. 1A & B illustrates small-angle X-ray scattering (SAXS) data of elastomeric lyotropic liquid crystal (E-LLC) and mesoporous elastomer showing that the mesoporous structure is intact after crosslinking. Full arrow indicated before and after crosslinking of the E-LLC gels, i.e. E-LLC gel (Fig 1A) and mesoporous elastomer (Fig. 1B).

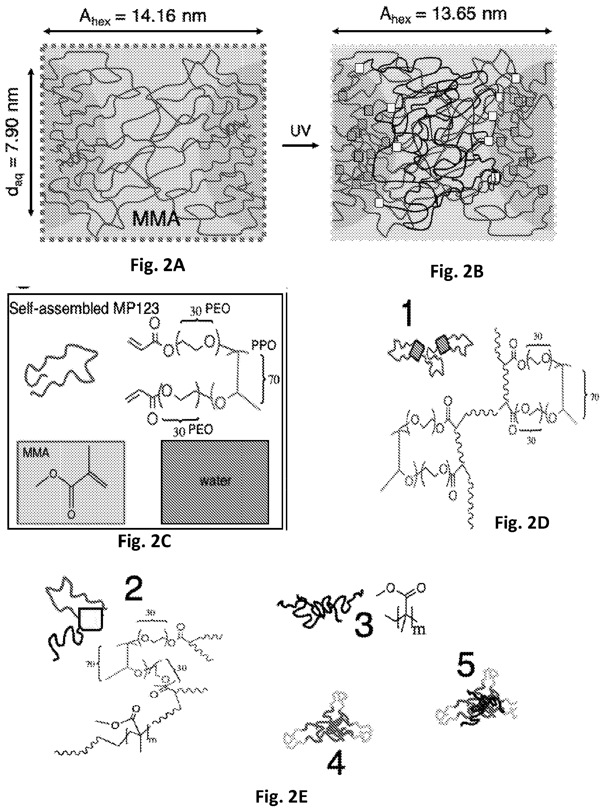

Fig. 2A,B,C,D & E illustrates synthesis scheme and crosslinking scheme of and within the mesoporous elastomer with a reverse hexagonal mesoporous structure. Reference 1 indicates MP123 covalently crosslinks with itself within the aqueous domains. Reference 2 indicates PMMA and MP123 covalently crosslink at the interface. Reference 3 indicates PMMA chains polymerize and covalently crosslink within the hydrophobic network. Reference 4 indicates hydrophobic PPO chains of the MP123 induce physical crosslinking. Reference 5 indicates hydrophobic PPO chains of the MP123 and long PMMA chains entangle and physically crosslink.

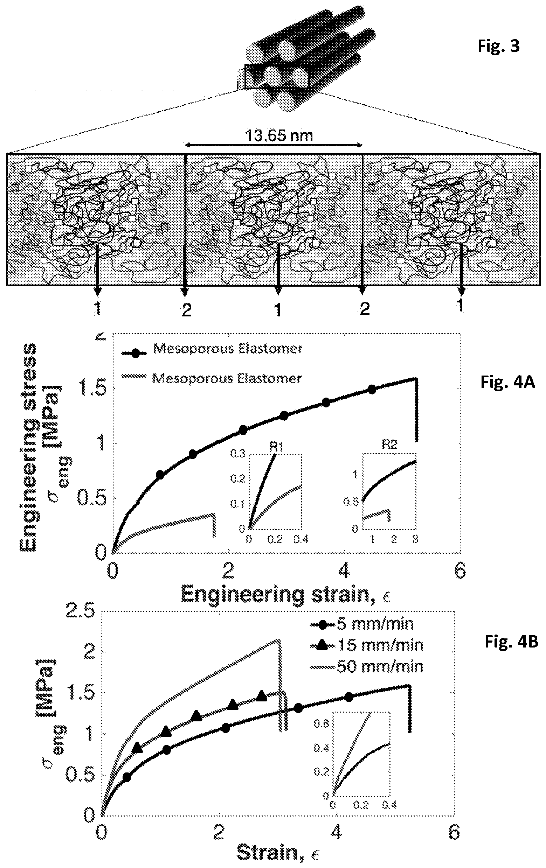

Fig. 3 illustrates periodically repeating physical and chemical crosslinks within the hydrophobic and hydrophilic domains, respectively, of the mesoporous elastomer. Reference number 1 represents a mixture of physical and chemical cross-links. Reference number 2 indicates pure covalent chemical cross-links.
Fig. 4A illustrates tensile testing of the mesoporous elastomer (line with circles) and comparing against a mesoporous elastomer without at least two of the required crosslinking types of both chemical and physical crosslinks (solid line). Fig. 4B shows strain stiffening of the mesoporous elastomer when elongated at different strain rates (solid line- 50 mm/min; line with triangles -15 mm/min; solid line - 50 mm/min).

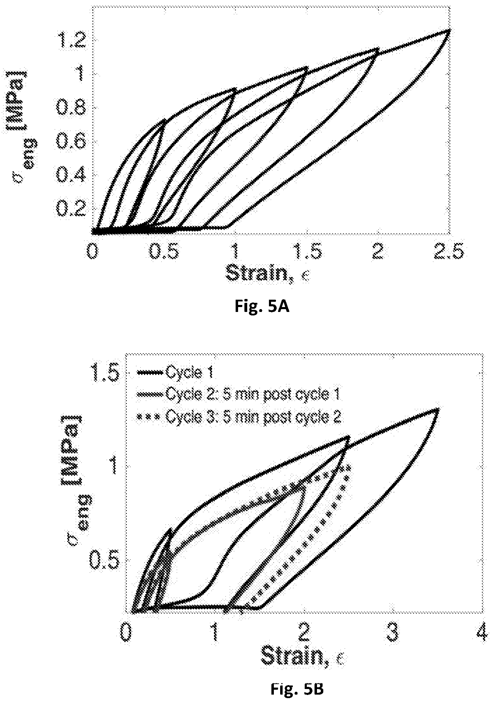

Fig. 5A illustrates cyclic tensile testing of the mesoporous elastomer and recovery efficiency of the mesoporous elastomer at continuous cyclic deformation. Fig 5B shows cyclic tensile testing of the mesoporous elastomer with specific time gaps between elongations.

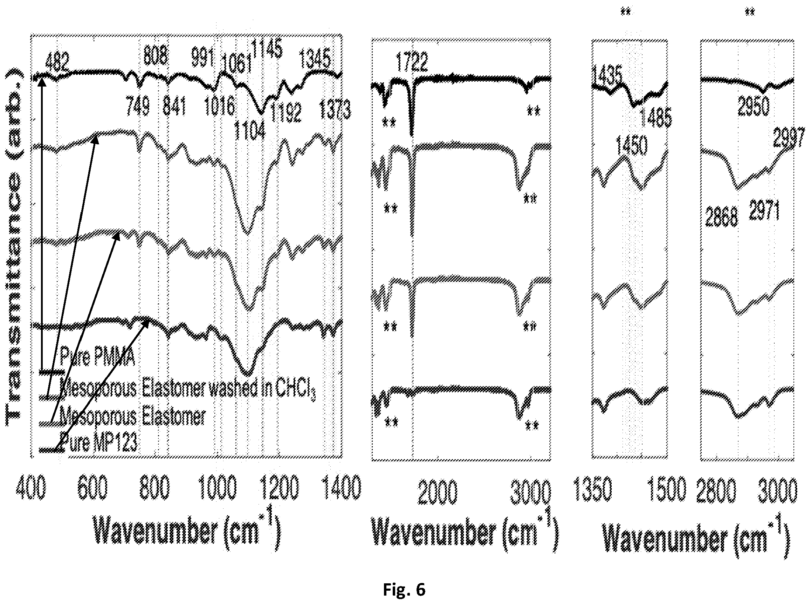

Fig. 6 illustrates Fourier transform infrared spectroscopy (FTIR) data of the mesoporous elastomer, mesoporous elastomer washed in CHCl₃, compared against pure diacrylate modified PLURONIC^{®} P123 (MP123) and pure poly(methyl methacrylate) (PMMA).

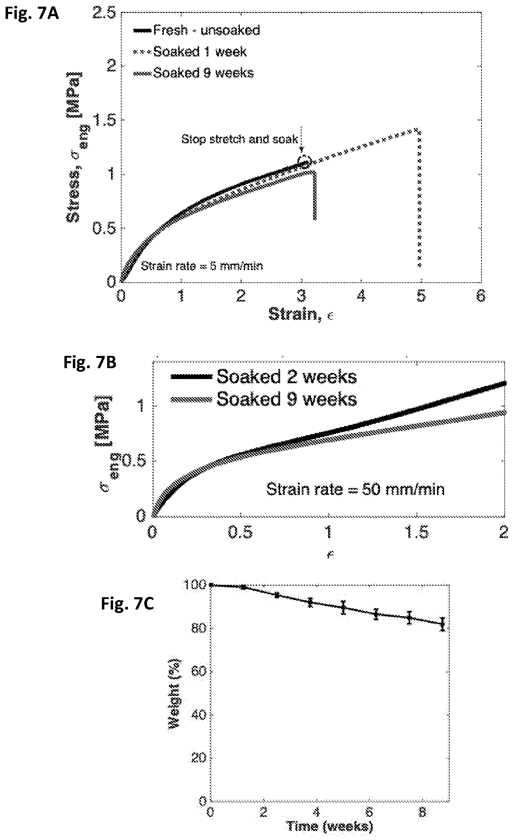

Fig. 7A illustrates mechanical stability of mesoporous elastomer when soaked in salt solutions at 37 °C for a maximum period of 9 weeks. Fig 7B shows mechanical stability of mesoporous elastomer soaked in salt solution for a maximum period of 9 weeks, at a strain rate of 50 mm/min. Fig 7C shows weight loss and degradation data of the mesoporous elastomer.

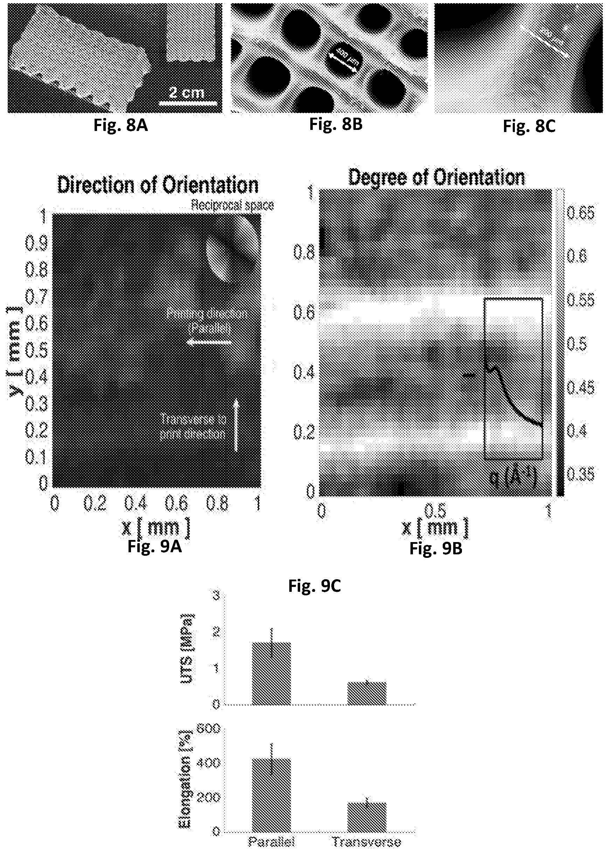

Fig. 8A, B & C shows digital photographs of the well- defined micro and macroscopic structures of the mesoporous elastomer.
Fig. 9A & B illustrates 2D scanning SAXS data of a region of the 3D printed mesoporous elastomers showing alignment of the mesoporous structure over the long-range. Fig 9C shows mechanical tensile testing data parallel and transverse/perpendicular to pore alignment leads to different behavior of the mesoporous elastomer. UTS is Ultimate Tensile Strength.

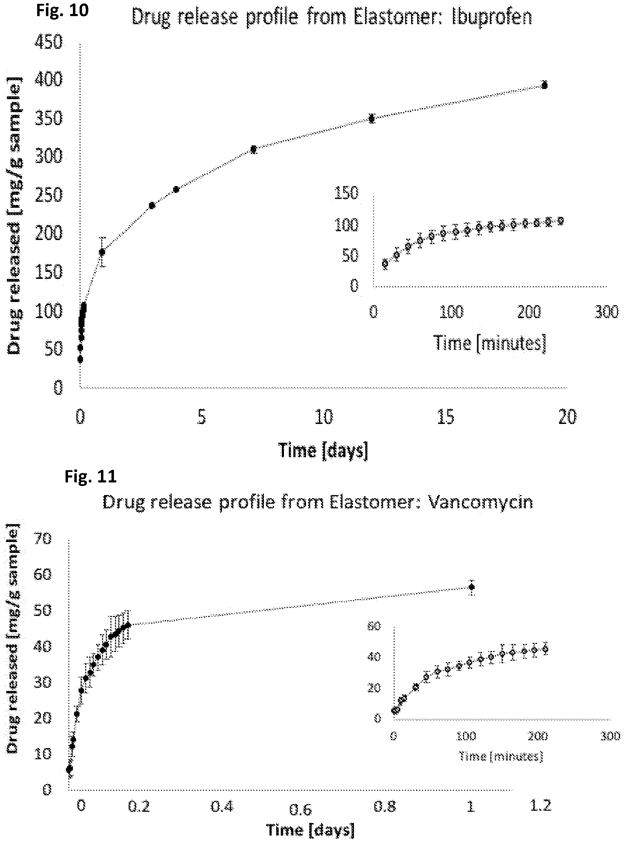

Fig. 10 is a graph showing sustained release of the hydrophobic anti-inflammatory drug Ibuprofen from the hydrophobic pores of the mesoporous elastomer.
Fig. 11 is a graph showing sustained release of the hydrophilic antibiotic drug Vancomycin from the hydrophilic pores of the mesoporous elastomer.

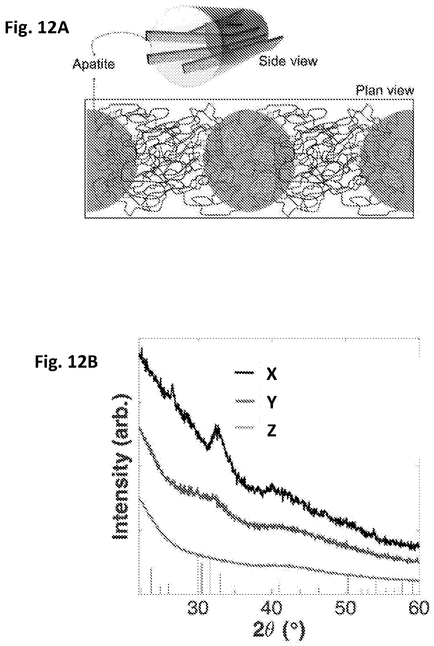

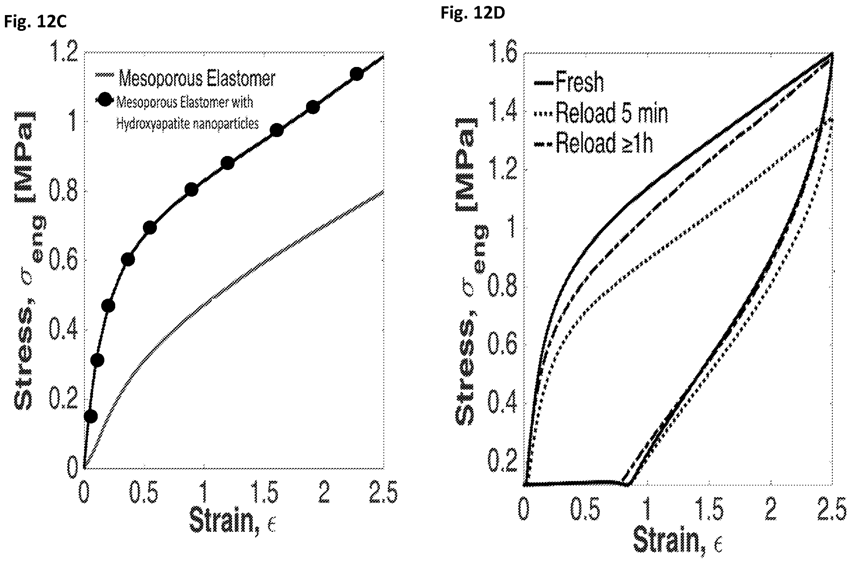

Fig. 12A is a schematic diagram showing location of hydroxyapatite nanoparticles in the hydrophilic pores of the mesoporous elastomer. Fig. 12B shows X-ray powder diffraction (XRD) data showing presence of hydroxyapatite nanoparticles within the mesoporous elastomer. Reference X corresponds to mesoporous elastomer with hydroxyapatite nanoparticles. Reference Y corresponds to mesoporous elastomer with amorphous calcium phosphate nanoparticles. Reference Z corresponds to mesoporous elastomer without nanoparticles, i.e. pure mesoporous elastomer. Fig. 12C shows tensile data of mesoporous elastomer-hydroxyapatite. Fig. 12D shows cyclic tensile data of mesoporous elastomer-hydroxyapatite materials.

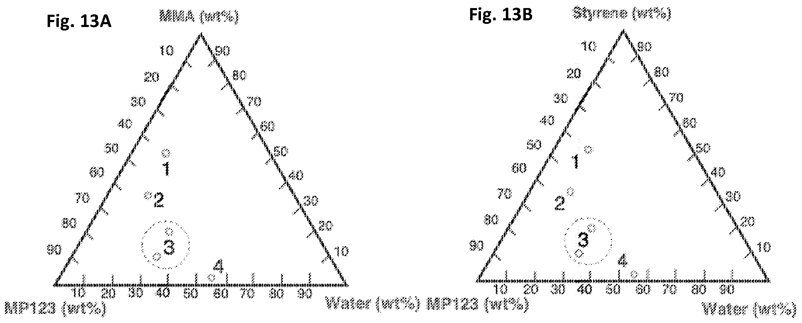

Fig. 13A is a three-phase diagram showing different compositions of MP123-water-methyl methacrylate to form a desired E-LLC of a specific mesoporous structure. Fig 13B shows a three-phase diagram showing the different compositions of MP123-water-styrene to form a desired E-LLC of a specific mesoporous structure.

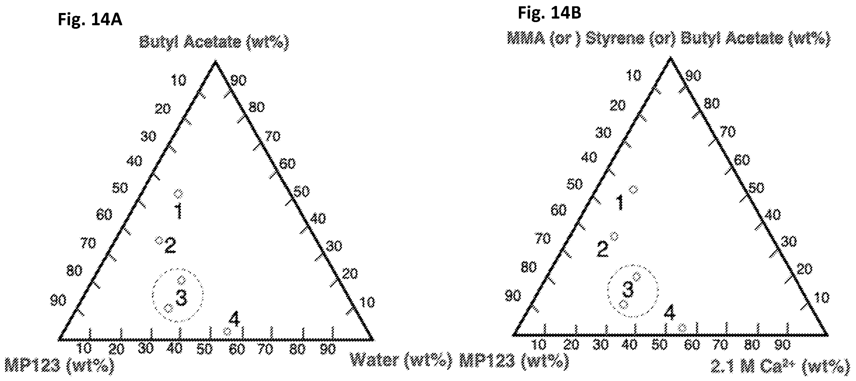

Fig. 14A is a three-phase diagram showing the different compositions of MP123-water-butyl acetate to form a desired E-LLC of a specific mesoporous structure. Fig 14B is a three-phase diagram showing the different compositions of MP123-2.1 M calcium phosphate precursor solution-desired hydrophobic liquid (MMA or styrene or butyl acetate) to form a desired E-LLC of a specific mesoporous structure. Reference 1 corresponds to E-LLC composition for a reverse micellar cubic mesoporous structure. Reference 2 corresponds to E-LLC composition for a reverse hexagonal mesoporous structure. Reference 3 corresponds to E-LLC composition for a lamellar mesoporous structure. Reference 4 corresponds to E-LLC composition for a normal hexagonal mesoporous structure

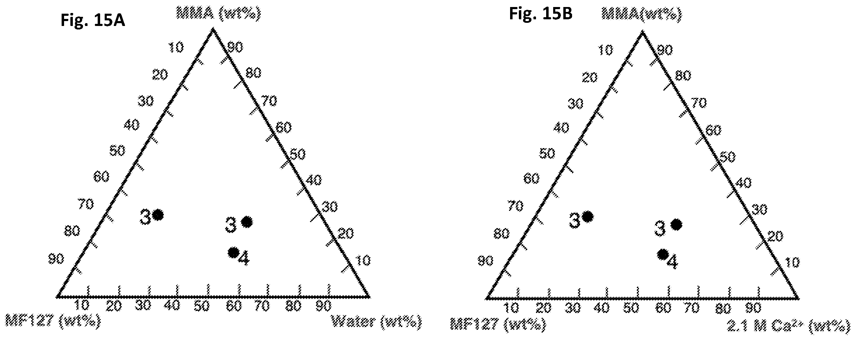

Fig. 15A is a three-phase diagram showing the different compositions of MF127-water-MMA to form a desired E-LLC of a specific mesoporous structure. Fig. 15B is a three-phase diagram showing the different compositions of MF127-2.1 M calcium phosphate precursor solution-MMA to form a desired E-LLC of a specific mesoporous structure. Reference 3 corresponds to E-LLC composition for a lamellar mesoporous structure. Reference 4 corresponds to E-LLC composition for a normal hexagonal mesoporous structure.

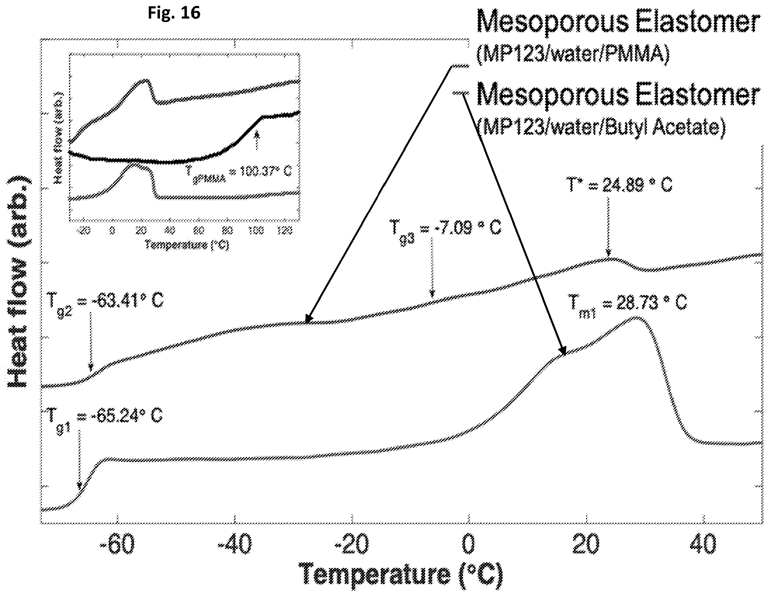

Fig. 16 is a differential scanning calorimetry plot of the Mesoporous Elastomer of Examples 1 and 2

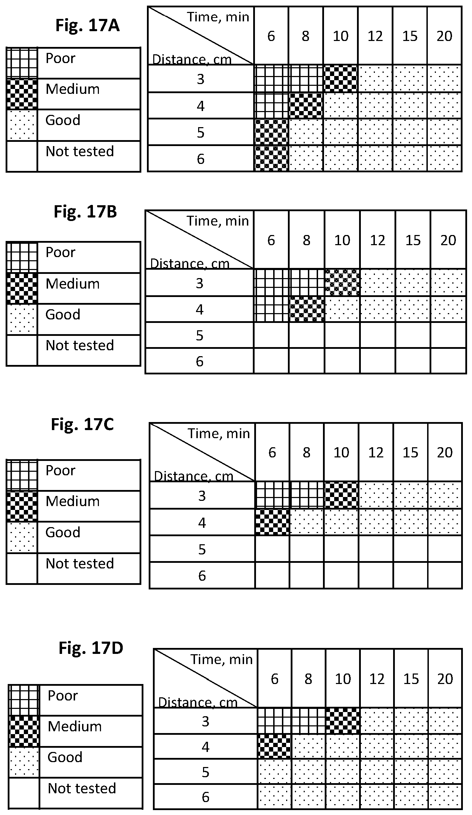

Fig. 17A, B, C & D is a qualitative map of the effect of three crosslinking parameters on the final crosslinked material by visually analyzing the mechanical feel of the mesoporous elastomer, for example, poor refers to gelly and soft and good refers to tough and elastic. The three parameters are UV exposure time, distance to UV lamp (i.e. intensity of light) and initiator concentration. The analysis was performed by visual evaluation. Fig. 17A shows a 4 wt% photoinitiator concentration. Fig. 17B shows a 1 wt% photoinitiator concentration. Fig. 17C shows a 2 wt% photoinitiator concentration. Fig. 17D shows a 0.4 wt% photoinitiator concentration.

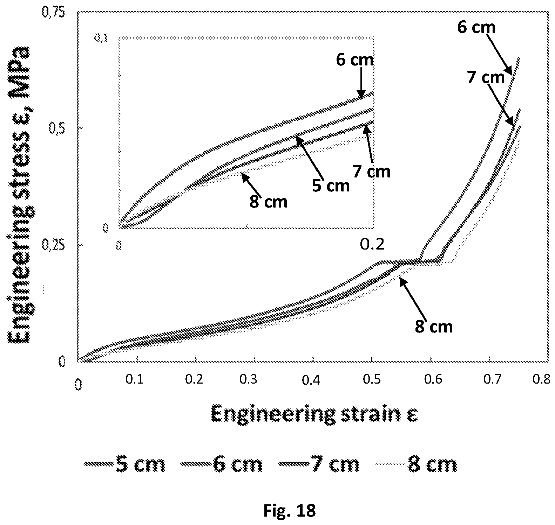

Fig. 18 is a graph showing stress-strain curves from a compression testing measurement of the mesoporous elastomer that was crosslinked at various distances from the UV lamp (that is, various light intensities) and at constant initiator concentration of 0.04 wt% and a constant UV exposure time of 10 min.

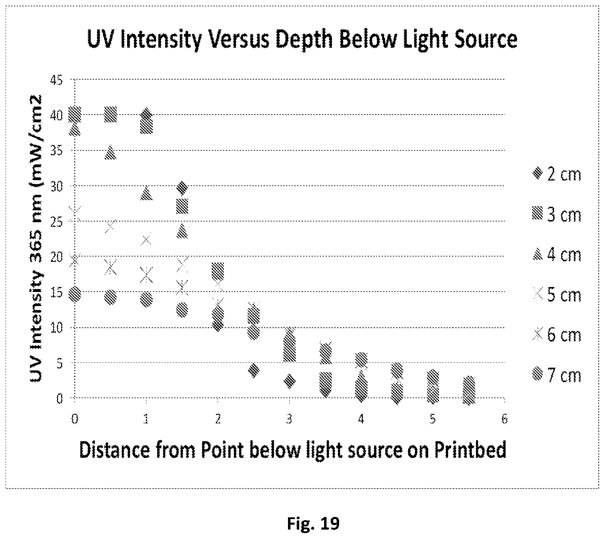

Fig. 19 is a graph showing UV intensity as a function of spot size and distance from UV lamp. Data provided by manufacturer, CELLINK AB, Sweden.

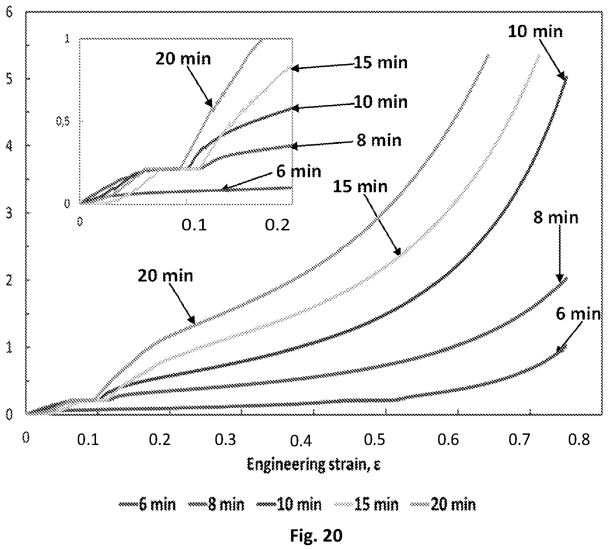

Fig. 20 is a graph showing stress-strain curves from a compression testing measurement of the mesoporous elastomer that was crosslinked using various UV exposure times by the UV lamp and at constant initiator concentration of 0.4 wt% and at a constant distance from UV lamp of 6 cm.

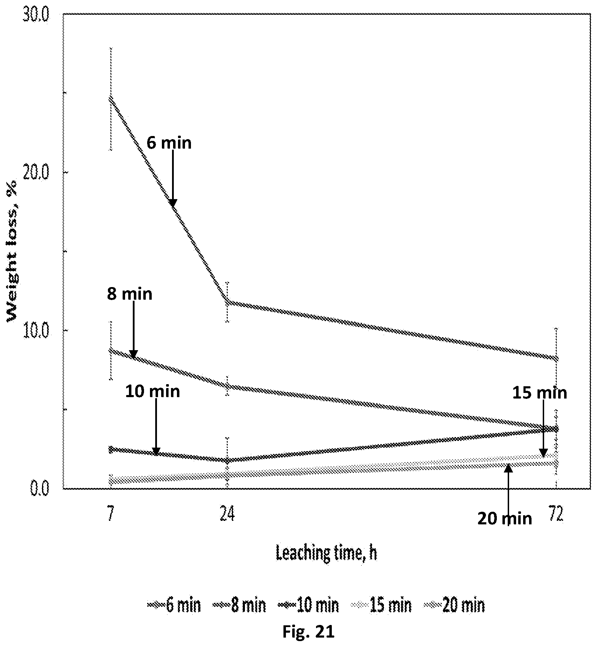

Fig. 21 is a graph showing mass loss curves from a leaching experiment where the residual weight of the mesoporous elastomer was measured after soaking in a phosphate buffer media. The mass loss of the mesoporous elastomer was measured against various UV exposure times by the UV lamp and at constant initiator concentration of 0.4 wt% and constant distance between sample and UV lamp of 6 cm.

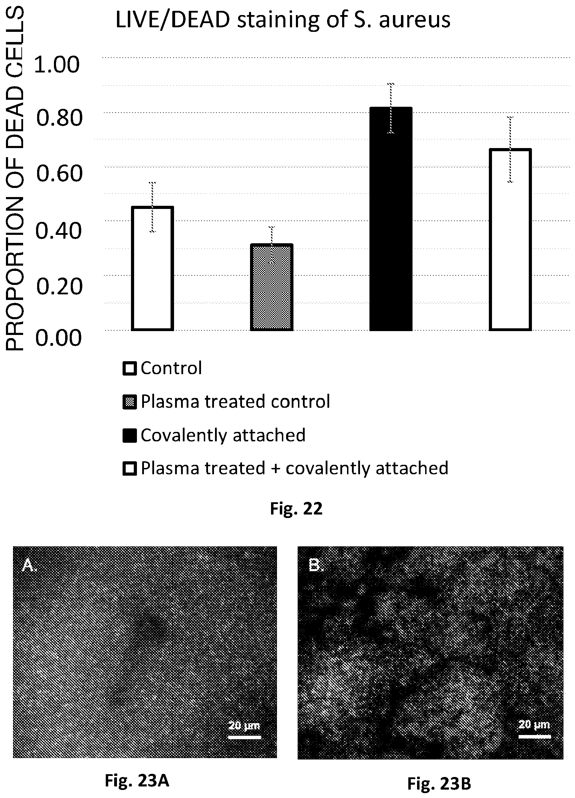

Fig. 22 shows a quantitative analysis of the proportion of dead bacterial cells (*Staphylococcus Aureus*) on the surface of control and covalently attached AMP mesoporous elastomer as shown in the Live/Dead Staining Images of Fig. 23. The proportion of dead cells was calculated using an image analysis macro according to the formula: (dead cells ÷ (dead cells + alive cells)) to account for differences in biofilm growth.
Fig. 23A shows live/dead images of various biofilms formed on a control mesoporous elastomer. Fig 23B shows live/dead images of biofilms on an antimicrobial mesoporous elastomer. Fig 23 shows *S*. *aureus.*

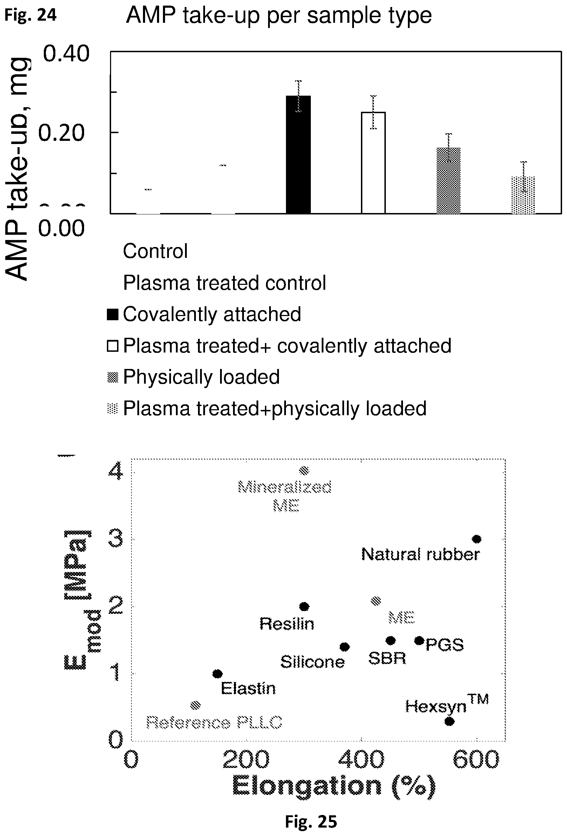

Fig. 24 is a graph showing uptake of AMP by the mesoporous elastomer when physically loaded and covalently attached together with varying the plasma treatment method as an added parameter. AMP uptake was measured using UV-Vis spectrometry.
Fig. 25 shows a comparison of E-modulus to elongation of the mesoporous elastomer (ME), a mineralized mesoporous elastomer (Mineralized ME), and a reference polymerized LLC (PLLC) with other elastomers.

### DETAILED DESCRIPTION

The present invention relates a multifunctional and mechanically tough elastomeric material with improved handling and with an amphiphilic, ordered and mesoporous structure, and to the production thereof. Despite great efforts at optimizing the molecular make-up of elastomers, little attention has been paid to designing their higher-level structural features at the nano-micro (meso) length scale, for example in the size range of 2-1000 nm. A well-defined mesostructure enables vital properties such as directional mechanics, multiscale toughening, improved tissue integration and therapeutic functionalities, such as local and sustained delivery of active substances. This is particularly relevant for elastomeric implants that are required to replace complex structured and mechanically vital biological materials, such as bone, skin, cartilage, muscle and blood vessels.

A controlled mesostructure with features, such as ordered nanoporous structure, monodisperse porosity and mesoscale anisotropy enables the formation of materials, which can possess direction dependent mechanical properties as well as chemically bind or physically host active substances that can be beneficial for an intended application. For example, release of a drug from a joint implant or endowing antimicrobial properties that can be valuable when considering the materials as catheters or wound dressings.

Liquid crystals (LCs) are polymeric materials that possess an ordered mesostructure and anisotropy and are currently used in a wide range of applications, such as electronics and membrane separation processes. One common type of LC polymers are known a lyotropic liquid crystalline (LLC) polymers (LLC) that are typically composed of a self-assembled amphiphilic molecule, also referred to as amphiphile herein, in a mixture of polar and non-polar solvents. LLCs usually possess an ordered mesoporous structure owing to the self-assembly characteristics of the amphiphilic molecule and the mesostructures can be in the form of spherical or cylindrical micelles arranged in cubic or hexagonal geometries, respectively. Another LLC mesostructure that can be formed is the lamellar sheet-like structure.

LLC-based polymers are generally held together via the hydrophobic interactions between amphiphilic molecules and the surrounding solvents. This results in the LLC polymers being mechanically weak gels or liquids with fluid-like behavior. In order to stabilize the mechanical integrity of the LLC polymers, covalent crosslinking has been applied wherein the amphiphilic molecules possess a crosslinkable functional group that enables crosslinking of all amphiphiles in the LLC to form a solid polymerized LLC (PLLC). An alternate route to form PLLCs has been via the formation of PLLC composites, which are prepared via the incorporation of additional fillers, such as a monomer, which can be either hydrophilic or hydrophobic, or an inorganic species, like nanoparticles, into the LLC system. By polymerizing the monomer within the LLC, a PLLC polymer can be prepared. In addition, PLLC composites have also been formed wherein a polymerizable LLC based on polymerizable amphiphiles is combined with a hydrophobic polymer to form nanoporous composites of PLLC and a filler material based on hydrophobic polymers. PLLC polymers, in general, have the advantage of being solid structures with an ordered nano-micro structure, such as monodisperse, and highly ordered nanoporosity and anisotropy.

Although solid structures, PLLC polymers are typically soft and difficult to handle after crosslinking. This leads to applications limited to substantially non load-bearing scenarios, such as for thin membrane coatings or as soft scaffolds for catalysis applications. Tough and mechanically robust PLLC-based elastomers with ordered structures have been prepared based on the chemical crosslinking of specialized amphiphilic molecules, such as polyisocyantes. While different PLLC polymers and elastomers have been developed for various engineering and electrical applications, their use in biomaterials has further been challenging due concerns relating to inherent toxicity of the component molecules and poor mechanical handling after crosslinking the LLC.

The present invention provides mesoporous elastomers, which are macroscopic, three-dimensional (3D) materials with high elasticity and stiffness that possesses an ordered and periodic nano-microporous, i.e., mesoporous, structure throughout the whole 3D macrostructure.

A mesoporous elastomer is a material that preferably satisfy one or all of the three criteria:
(1) possesses an ordered arrangement of pores with monodisperse size that can be in range of 1-1000 nm, such as 2-1000 nm, throughout the 3D space of the material;
(2) possesses a combination of covalent chemical crosslinks and physical chain entanglement crosslinks that mechanically stabilize and improve the handling of the mesoporous elastomer; and
(3) displays highly elastic deformation, for example, in the range between 50-1000% tensile elongation and between 50-100% compressive deformation and stiffness in range of 0.1-20 MPa.

Based on the above criteria, an embodiment of the mesoporous elastomer may be an elastomeric LLC (E-LLC) based elastomeric material that is formed by a combination of covalent chemical crosslinking and physical chain entanglement crosslinking at the molecular and nanostructural level to endow 2D and 3D macroscopic materials with high elasticity and stiffness and with an ordered mesoporous structure. An ELLC-based mesoporous elastomer is essentially a singular material at the molecular and nanostructural level and is not a composite material.

An ELLC-based mesoporous elastomer is formed via the molecular self-assembly and intermolecular crosslinking of polymerizable amphiphilic molecules and a mixture of hydrophilic and hydrophobic solvents. The polymerizable amphiphilic species for forming the mesoporous elastomer can be any amphiphilic molecule that preferably meet the following characteristics:
(1) possesses one or more hydrophilic parts as well as one or more hydrophobic parts in the same molecule;
(2) possesses a polymerizable group on either the hydrophilic or hydrophobic or on both parts; and
(3) self-assembles into LLC micellar architectures, such as cubic, hexagonal or lamellar structures, in the presence of solvent mixtures.

Non-limiting, but illustrative, examples of a polymerizable group according to requirement (2) above could be an acrylate group or an amide group on the hydrophilic part of the amphiphilic molecule that can chemically, i.e., covalently, crosslink with neighboring amphiphilic molecules.

The solvent mixtures for forming a mesoporous elastomer can be purely hydrophilic in nature or purely hydrophobic in nature or a mixture of hydrophilic and hydrophobic species. Thus, in a preferred embodiment the mesoporous elastomer is based on a mixture of; polymerizable amphiphilic molecules and hydrophilic and hydrophobic solvents. In other embodiments, the mesoporous elastomer is based on a mixture of; polymerizable amphiphilic molecules and a hydrophilic solvent or, a mixture of; polymerizable amphiphilic molecules and a hydrophobic solvent. Moreover, either the hydrophilic solvent or the hydrophobic solvent or both solvents can be polymerizable monomers by themselves, such that those species can also crosslink chemically or physically with the amphiphilic molecules and with each other.

In a preferred embodiment of the invention, the mesoporous elastomer is formed using polymerizable amphiphilic molecules, hydrophilic solvents and hydrophobic solvents, all of which are preferably non-toxic to human cells.

In a preferred embodiment of the invention, a mesoporous elastomer possesses a reverse hexagonally ordered mesostructure with cylindrical pores of 1-1000 nm, such as 2-1000 nm, preferably 2-100 nm, such as about 10 nm, and is formed by mixing a polymerizable amphiphile, the hydrophilic solvent, such as water or an aqueous solvent, and the hydrophobic solvent, such as a hydrophobic monomer solvent, wherein the hydrophobic monomer solvent can also act as a polymeric monomer.

In a particular embodiment, the polymerizable amphiphile is a polymerizable triblock copolymer. Non-limiting, but illustrative, examples of such polymerizable triblock copolymers are diacrylate modified PLURONIC^{®}. PLURONIC^{®} is a trade name by BASF Corporation for amphiphilic copolymers with a central polymer chain of polypropylene oxide flanked by side chains of polyethylene oxide:

HO-(CH₂CH₂O)ₙ-(CH₂CHCH₃O)ₘ-(CH₂CH₂O)ₚ-H

In an embodiment, n is preferably equal to p. Table 1 below lists various PLURONIC^{®} copolymers.

**Table 1 - PLURONIC^{®} copolymers**

| Name | Average molecular weight (g/mol) | Viscosity (cps) | Solubility in H₂O at 25 °C (%) | Melt point (°C) |
|---|---|---|---|---|
| L31 | 1 100 | 175* | >10 | - |
| L35 | 1 900 | 375* | >10 | - |
| L43 | 1 850 | 310* | >10 | - |
| L44 | 2 200 | 440* | >10 | - |
| L61 | 2 000 | 325* | insoluble | - |
| L62 | 2 500 | 450* | >10 | - |
| L64 | 2 900 | 850* | >10 | - |
| L81 | 2 750 | 475* | insoluble | - |
| L92 | 3 650 | 700* | >1 | - |
| L101 | 3 800 | 800* | insoluble | - |
| L121 | 4 400 | 1 200* | insoluble | - |
| P65 | 3 400 | 180** | >10 | - |
| P84 | 4 200 | 280** | >10 | - |
| P85 | 4 600 | 310** | >10 | - |
| P103 | 4 950 | 285** | >10 | - |
| P104 | 5 900 | 390** | >10 | - |
| P105 | 6 500 | 750* | >10 | - |
| P123 | 5 750 | 350** | >10 | - |
| F38 | 4 700 | 260*** | >10 | 48 |
| F68 | 8 400 | 1 000*** | >10 | 52 |
| F77 | 6 600 | 480*** | >10 | 48 |
| F87 | 7 700 | 700*** | >10 | 49 |
| F88 | 11 400 | 2 300*** | >10 | 54 |
| F98 | 1 300 | 2 700*** | >10 | 58 |
| F108 | 14 600 | 2 800*** | >10 | 57 |
| F127 | 12 600 | 3 100*** | >10 | 56 |

| | | | | |
|---|---|---|---|---|
| * Viscosity [Brookfield] at 25 °C ** Viscosity [Brookfield] at 60 °C *** Viscosity [Brookfield] at 77 °C | | | | |

Particular preferred PLURONIC^{®} copolymers include diacrylate modified PLURONIC^{®} P123 (MP123), diacrylate modified PLURONIC^{®} F127 (MF127) and diacrylate modified PLURONIC^{®} L64 (ML64).

Other examples of polymerizable amphiphilic molecules that can be used according to the embodiments include polymerizable surfactants, such as diacrylate modified Brij-S10 (MBrij S-10).

In a particular embodiment, the hydrophobic solvent is a non-polar solvent or liquid, such as a hydrophobic monomer solvent, e.g., methyl methacrylate (MMA), hexanediol diacrylate, isoprene, isobutylene, urethanes, chloroform, styrene, or butyl acetate.

In a particular embodiment, the hydrophilic solvent is a polar solvent or liquid, such as water, an aqueous solution; a liquid, solvent, colloid or suspension comprising or consisting of hydrophilic monomers, such as ethanol and water miscible monomers, e.g., hydroxyethyl methacrylate (HEMA).

Table 2 below lists examples of various hydrophilic and hydrophobic solvents that can be used according to the embodiments, including suggested composition and resulting mesoporous structure.

**Table 2 - composition examples and type of mesoporous structure**

| Polymerizable amphiphile | Hydrophilic solvent | Hydrophobic solvent | Composition (wt%, polymerizable amphiphile: hydrophilic:hydrophobic) | Mesoporous structure |
|---|---|---|---|---|
| MP123 | Water | MMA | 50:15:35 | Reverse Hexagonal |
| MP123 | Water | Styrene | 50:15:35 | Reverse Hexagonal |
| MP123 | 2.1 M Ca²⁺ Solution | MMA | 50:15:35 | Reverse Hexagonal |
| MF127 | Water | MMA | 35:50:15 | Normal Hexagonal |
| MF127 | Water | Styrene | 35:50:15 | Normal Hexagonal |
| MF127 | 2.1 M Ca²⁺ Solution | MMA | 35:50:15 | Normal Hexagonal |
| MP123 | Water | MMA | 30:60:10 | Lamellar |
| MP123 | Water | MMA | 50:30:20 | Lamellar |
| MP123 | Water | MMA | 35:12.5:52.5 | Reverse Micellar Cubic |
| MP123 | 2.1 M Ca²⁺ Solution | MMA | 30:60:10 | Lamellar |
| MP123 | 2.1 M Ca²⁺ Solution | MMA | 50:30:20 | Lamellar |
| MP123 | 2.1 M Ca²⁺ Solution | MMA | 35:12.5:52.5 | Reverse Micellar Cubic |
| MP123 | Water | Butyl Acetate | 30:60:10 | Lamellar |
| MP123 | Water | Butyl Acetate | 50:30:20 | Lamellar |
| MP123 | Water | Butyl Acetate | 35:12.5:52.5 | Reverse Micellar Cubic |
| MP123 | 2.1 M Ca²⁺ Solution | Butyl Acetate | 30:60:10 | Lamellar |
| MP123 | 2.1 M Ca²⁺ Solution | Butyl Acetate | 50:30:20 | Lamellar |
| MP123 | 2.1 M Ca²⁺ Solution | Butyl Acetate | 35:12.5:52.5 | Reverse Micellar Cubic |

On mixing the three components at specific ratios, a reverse hexagonally ordered LLC is formed, in which the amphiphile self-assembles into cylindrical micelles in a hexagonal geometry with water enclosed within the cylinders and the MMA molecules occupy the space outside the cylinders. In effect, the LLC structure can be imagined as the arrangement of water molecules and MMA molecules in an alternating and periodic manner uniformly separated by the nanoscale micellar cylinders formed by the triblock polymerizable amphiphile. Subsequently, at the macroscale, the mixture forms a thick viscous liquid that might be referred to as the precursor to the mesoporous elastomer, referred to as an elastomeric lyotropic liquid crystal (E-LLC) herein. The E-LLC is then transformed into the singular and homogenous solid mesoporous elastomer in such a way that all three components, e.g., water, polymerizable amphiphile and MMA crosslink through a combination of chemical covalent crosslinking as well as physical chain entanglement crosslinking.

In the above particular embodiment, the chemical covalent crosslinking in the mesoporous elastomer is achieved via at least one pathway and preferably at least two pathways and most preferably via more than two pathways, such as all three pathways for chemical covalent crosslinking presented below. The following chemical covalent crosslinking pathways or scenario can occur individually as well as in coordination:
(1) Homogenous chemical crosslinking of the polymerizable triblock amphiphiles with neighboring polymerizable triblock amphiphiles;
(2) Interfacial and heterogeneous chemical crosslinking of the polymerizable triblock amphiphiles with the hydrophobic MMA monomers that further form poly MMA (PMMA) chains; and
(3) Homogenous chemical crosslinking of MMA monomers to form long polymeric crosslinked chains of PMMA.

In a general case, the following chemical covalent crosslinking pathways or scenario can occur individually as well as in coordination:
(1) Homogenous chemical crosslinking of the polymerizable amphiphiles with neighboring polymerizable amphiphiles;
(2) Interfacial and heterogeneous chemical crosslinking of the polymerizable amphiphiles with hydrophobic monomers that further form hydrophobic polymer chains and/or interfacial and heterogeneous chemical crosslinking of the polymerizable amphiphiles with hydrophilic monomers that further form hydrophilic polymer chains; and
(3) Homogenous chemical crosslinking of hydrophobic monomers to form long polymeric crosslinked hydrophobic polymer chains and/or homogenous chemical crosslinking of hydrophilic monomers to form long polymeric crosslinked hydrophilic polymer chains.

In the above particular embodiment, the physical chain entanglement crosslinking in the mesoporous elastomer is achieved by at least one pathway and preferably at least two pathways and most preferably by more than two pathways, such as all three pathways for physical chain entanglement crosslinking presented below. The following physical chain entanglement crosslinking pathways or scenarios can occur individually as well as in coordination:
(1) Homogenous physical chain entanglements of the hydrophobic parts of the polymerizable triblock amphiphiles;
(2) Homogenous chain entanglements of the long polymeric chains of PMMA molecules; and
(3) Heterogeneous physical chain entanglements between the long polymeric PMMA chains and the hydrophobic parts of the polymerizable triblock amphiphiles.

In a general case, the following physical chain entanglement crosslinking pathways or scenarios can occur individually as well as in coordination:
(1) Homogenous physical chain entanglements of the hydrophobic parts of the polymerizable amphiphiles;
(2) Homogenous chain entanglements of the long hydrophobic polymeric chains and/or homogenous chain entanglements of the long hydrophilic polymeric chains; and
(3) Heterogeneous physical chain entanglements between the long hydrophobic polymeric chains and the hydrophobic parts of the polymerizable amphiphiles and/or heterogeneous physical chain entanglements between the long hydrophilic polymeric chains and the hydrophilic parts of the polymerizable amphiphiles.

In general, the mesoporous elastomer is characterized by the presence of both chemical covalent crosslinking and physical chain entanglement crosslinking, which is necessary for the improved handling and mechanical stability of the mesoporous elastomer. Absence of any one crosslinking system, i.e., chemical covalent crosslinking or physical chain entanglement crosslinking, will compromise the handling and mechanical properties. The present invention is built on the highly coordinated and combinatorial effect of both crosslinking systems for the formation of the mesoporous elastomer.

In the aforementioned embodiments of the mesoporous elastomer, the amphiphilic molecule can be chosen from a group of amphiphilic molecules that can be chemically and physically crosslinked and shows the tendency to form E-LLC with hydrophilic, such as polar, and hydrophobic, such as non-polar, solvents. The hydrophilic solvents can be either water, an aqueous solvent or any hydrophilic monomers and/or polymers that can be incorporated together with water or the aqueous solvent. Such hydrophilic monomers and/or polymers include hydroxyethyl methacrylate (HEMA), acrylamide, polyacrylamide, and alginate. The hydrophobic solvent can be any hydrophobic solvent, which includes hydrophobic monomers and/or polymers, comprising, but not limited to MMA, acrylates, methacryaltes, isobutylenes, isoprenes, urethanes, aramids and styrene.

In another aspect, the invention also provides a method to form or produce a mesoporous elastomer with a well-defined mesoporous structure via the formation and crosslinking of a so-called elastomeric lyotropic liquid crystal (E-LLC).

The E-LLC precursor is formed by mixing together:
(a) polymerizable amphiphile, preferably with capabilities to crosslink chemically via reactive chemical bonds and physically via chain entanglements;
(b) a hydrophilic solvent, such as water, an aqueous solution or hydrophilic monomers and/or polymers; and
(c) a hydrophobic solvent, such as a non-polar solvent or hydrophobic monomers and/or polymers.

In an embodiment, these three species are included to form the E-LLC with a mesoporous structure. The particular mesoporous structure obtained depends on the respective amounts of the three species as is further disclosed herein

In an embodiment, the E-LLC is crosslinked using, for instance, light or heat or via chemical means or a combination thereof, to form the, preferably solid, mesoporous elastomer such that the mesoporous elastomer is comprised of a combination of covalent chemical crosslinks and physical chain entanglement crosslinks.

The mesoporous elastomer is substantially amphiphilic due to its LLC character, such that the pores of the mesoporous elastomer are able to display both hydrophilic and hydrophobic behavior. For example, some pores of the mesoporous elastomer are hydrophilic and can display absorption of hydrophilic liquids, such as water, salt solutions, hydrophilic monomers, etc., whereas other pores of the same mesoporous elastomer are hydrophobic and can display absorption of hydrophobic liquids, including solvents, monomers and polymers.

The mesoporous elastomer, owing to its amphiphilic nature, can bind chemical agents of hydrophilic nature and chemical agents of hydrophobic nature, such as drugs or any organic or inorganic materials within its mesopores. The binding between such agents and the pores of the mesoporous elastomer can be of chemical interaction, such as covalent linkages, or via physical interactions e.g., spatial encapsulation.

The mesoporous elastomer displays high elasticity and stiffness under tensile deformation. The mesoporous elastomer can display rapid elastic recovery when deformed from 50% elongation to 1000% elongation without rupture or failure. Average stiffness of a mesoporous elastomer according to the embodiments can be in the range of 0.1-20 MPa. The mesoporous elastomer is fatigue resistant in that the materials resist cyclic deformation for at least 10 cycles.

An advantage of this invention is the high elasticity and stiffness that arises out of the molecular crosslinked network of the mesoporous elastomer. One such example is that the physical chain entanglement crosslinking of the mesoporous elastomer resists tensile deformation at low strain rates, for example <100% elongation, and the covalent chemical crosslinking of the mesoporous elastomer resists deformation >100% elongation. The dualistic mechanical response of the mesoporous elastomer is a direct effect of its molecular crosslinking and periodic mesoporous structure.

The mesoporous elastomer with various other mesoporous structures can also be formed using E-LLC precursors possessing ordered mesoporous structures other than the cylindrical reverse hexagonal LLC geometry. Other possible mesoporous structures include normal micellar, micellar cubic, normal hexagonal, lamellar, reverse micellar cubic, cubic bicontinous and reverse micellar.

Generally, in the presence of hydrophilic (polar) and hydrophobic (non-polar) solvents, an amphiphilic molecule self-assembles to form nanostructures called micelles. Depending on the concentration of each species, the micelles can be present in different shapes, such as cylindrical, spherical or lamellar sheet assemblies. In such a system, the polar solvent, such as water, can be present outside the micelle and the non-polar solvent can be located inside the micelle, or vice versa. The amphiphilic molecule usually acts as the interface between the two solvents. Groups of these micellar assemblies that are arranged in a specific, long-range and periodic geometry are called lyotropic liquid crystals (LLCs). When the LLC can be chemically and physically crosslinked to give a tough mesoporous elastomer, it can be classified as an E-LLC.

The self-assembled nanostructure of the E-LLCs can be of various geometries, such as type 1 systems and type 2 systems detailed below and as further demonstrated in the ternary phase diagrams in Figs. 13-15.

Type 1: Micellar, vesicular and E-LLC nanostructures that contain polar solvent, such as water, as the continuous domain and non-polar solvent is confined within the confined regions of micelles, vesicles or LLCs.
(1) Spherical micellar aggregates in size range of 2-100 nm arranged randomly throughout a liquid suspension called as a normal micellar system;
(2) Spherical micellar aggregates in the size range 2-100 nm arranged as an E-LLC, which is cubic shaped, ordered arrangement known as a normal micellar cubic system with Im3m crystal structure;
(3) Spherical micellar aggregates in the size range 2-100 nm arranged as an E-LLC, bicontinuous cubic shaped, ordered arrangement known as a normal micellar cubic system with P63m crystal structure;
(4) Spherical micellar aggregates in the size range 2-100 nm arranged as an E-LLC, bicontinuous cubic shaped, ordered arrangement known as a normal micellar cubic system with la3d crystal structure;
(5) Cylindrical micellar aggregates with diameter of cylinders in the size range of 2-100 nm arranged as an ordered E-LLC, hexagonal geometry called as a normal hexagonal system; and
(6) Lamellar sheet-like micellar aggregates with sheet thickness of 2-100 nm arranged as an ordered E-LLC called as lamellar system.

Type 2: Micellar and E-LLC nanostructures that can contain organic monomer as the continuous domain and polar solvent, such as water, is confined within the micellar aggregates.
(1) Spherical micellar aggregates in size range of 2-100 nm arranged randomly called as a reverse micellar system;
(2) Spherical micellar aggregates in the size range 2-100 nm arranged as an E-LLC, cubic shaped, ordered arrangement known as a reverse micellar cubic system with Im3m crystal structure;
(3) Spherical micellar aggregates in the size range 2-100 nm arranged as an E-LLC, bicontinuous cubic shaped, ordered arrangement known as a reverse micellar cubic system with P63m crystal structure;
(4) Spherical micellar aggregates in the size range 2-100 nm arranged as an E-LLC, bicontinuous cubic shaped, ordered arrangement known as a reverse micellar cubic system with la3d crystal structure; and
(5) Cylindrical micellar aggregates with diameter of cylinders in the size range of 2-100 nm arranged as an E-LLC, hexagonal geometry called as a reverse hexagonal system.

An embodiment of the invention relates to injectable or 3D printable mesoporous elastomers that might be locally injected or 3D printed as the E-LLC into any desired shape and form and crosslinked into a solid mesoporous elastomer with high elasticity and improved handling and possessing an ordered and well-defined mesoporous structure.

Another embodiment of the mesoporous elastomer is, but not limited to, medical device applications. One such application is the urinary catheters with ordered mesoporosity throughout its entire 3D structure. Another application is injectable joint implants where injectability and mesoporosity is combined for the necessary application.

A further embodiment of the mesoporous elastomer is the formation of mesoporous elastomeric composites, where the mesoporous elastomer can be formed with inorganic species at the nanostructural level to form ordered and mesostructured mesoporous elastomer composites for further improved handling and increased stiffness.

The mesoporous elastomer of the embodiment is substantially non-biodegradable or might be considered to be slowly degradable over a period greater than 1 year.

In an embodiment, the mesoporous elastomer is formed by first synthesizing a chemically and physically crosslinkable precursor material called E-LLC. The E-LLC can be, but is not limited to, a non-viscous liquid dispersion or a viscous gel. The term crosslinkable (or polymerizable) refers to the ability to chemically crosslink (or polymerize) and physically crosslink the molecules or monomers or polymers of the E-LLC to form a dense, crosslinked mesoporous elastomer. The precursor to the mesoporous elastomers, i.e., the E-LLC, is a specific chemical composition of molecules that can form self-assembled dispersions like crosslinkable (or polymerizable) micellar solutions and self-assembled viscous liquids like crosslinkable (or polymerizable) LLCs of a defined mesoporous structure. The mesoporous structures of an E-LLC can be of various geometries as described above.

In an embodiment, an E-LLC is an elastomeric LLC that comprises at least two types of chemical species and more preferably three types of chemical species, which when mixed together form a non-viscous liquid-like dispersion or viscous gel that possesses an ordered and periodic mesoporous structure throughout its entire macroscopic volume. The different chemical species in an E-LLC can be crosslinked both chemically via covalent chemical crosslinks and physically via chain entanglements to form the final mesoporous elastomer. The combination of chemical and physical crosslinks is vital for the handling and toughness of the mesoporous elastomer produced from the E-LLC.

An E-LLC can be formed by the molecular self-assembly of crosslinkable (or polymerizable) amphiphilic molecules, such as crosslinkable surfactants, e.g., diacrylate modified polyoxyethylene (10) stearyl ether (or diacrylate modified Brij S-10), diacrylate modified cationic surfactants, such as diacrylate modified cetyltriethyl ammonium bromide, crosslinkable block copolymers, e.g., diacrylate modified polyethylene oxide-polypropylene oxide triblock copolymers, such diacrylate modified PLURONIC^{®} P123, diacrylate modified PLURONIC^{®} F127, diacrylate modified PLURONIC^{®} L64 and diacrylate modified PLURONIC^{®} L121, crosslinkable proteins, e.g., crosslinkable collagen derivatives, such as gelatin methacrylate, crosslinkable peptides or crosslinkable lipids, e.g., diacrylate modified glycerol monoleate, diyne phospholipids, in the presence of at least one of a hydrophilic solvent, such as water, and a hydrophobic solvent, such as organic solvents. Hydrophilic, such as polar, solvents can be water, any aqueous solutions or any hydrophilic solvent that might experience dipole moments within itself. Hydrophobic, such as non-polar, solvents can be hydrophobic solvents, such as chloroform, butyl acetate or hydrophobic monomers and/or polymers, such as methyl methacrylate or styrene. The hydrophobic solvent is preferably either insoluble or only sparingly soluble with the hydrophilic solvent. The type and composition of hydrophilic solvent, hydrophobic solvent and amphiphilic molecule used to form the E-LLC should be selected in such a way that the mixture of the three components results in an E-LLC that possesses an ordered mesoporous structure of defined geometry as detailed above. An example of an E-LLC is a reverse hexagonal mesoporous E-LLC gel that comprises, preferably consists of, the three-phase mixture of a polymerizable amphiphilic triblock copolymer, water and a hydrophobic monomer in terms of methyl methacrylate (Example 1).

In an embodiment, a mesoporous elastomer is an elastomeric solid material that is molecularly crosslinked via a combination of covalent chemical and physical chain entanglement crosslinks and comprises mesopores of the size range 2-1000 nm and exhibits mechanically tough and elastic character. A mesoporous elastomer is formed via the covalent chemical and physical chain entanglement crosslinking of the E-LLC precursor. After crosslinking, the mesoporous structure of the E-LLC is preferably not disturbed or disrupted in any manner, or in other words, the mesoporous structure of the E-LLC is preferably exactly intact in the resulting crosslinked mesoporous elastomer (SAXS data in Fig. 1). The E-LLC can be of various mesoporous structures depending upon the concentrations of the chemical species present within the system. For example, an E-LLC with reverse hexagonal mesoporous structure comprises the polymerizable amphiphile and a hydrophobic monomer as the majority species (>60 wt%) and water as the minority species (<30 wt%). The mesoporous aspect of the mesoporous elastomer can be defined as the mesoporous elastomer having a porous structure where the pore sizes are monodispersed and can range in the size regime of 2-1000 nm. The pore geometry can further be tuned to possess a cylindrical geometry as derived from a reverse hexagonal E-LLC or bilayer geometry as derived from a lamellar E-LLC or a continuous cubic geometry as derived from a micellar cubic E-LLC (Table 3).

The covalent chemical crosslinking of E-LLCs can be achieved by means of light, heat or catalysts or a combination thereof. The chemical crosslinking of the E-LLC can be facilitated by at least two of the three possible chemical reactions listed below:
(1) The E-LLC is crosslinked using crosslinkable chemical groups present on the hydrophilic head and/or hydrophobic tail on the crosslinkable (or polymerizable) amphiphilic molecules of the E-LLC;
(2) The E-LLC can be crosslinked using crosslinkable (or polymerizable) chemical groups on the amphiphilic monomer and together with the crosslinkable chemical groups in/on the hydrophobic monomer present within the hydrophobic domains (pores) of the E-LLC and/or using crosslinkable (or polymerizable) chemical groups on the amphiphilic monomer and together with the crosslinkable chemical groups in/on the hydrophilic monomer present within the hydrophilic domains (pores) of the E-LLC; and
(3) The E-LLC is chemically crosslinked via the polymerization and chemical crosslinking of the hydrophobic monomer within the hydrophobic domains (or pores) (Fig. 2) and/or via the polymerization and chemical crosslinking of the hydrophilic monomer within the hydrophilic domains (or pores).

Common functional groups that are preferably present to achieve covalent chemical crosslinking in the E-LLC using any of the above methods include, but are not limited to, vinyl groups that can form alkyl linkages (-C=C end groups to -C-C- linkages) or carboxyl-amine reactive sites that can form amide linkages (-C=O and NH₂ end groups to give -CONH- linkages). These groups can be present solely on the crosslinkable (or polymerizable) amphiphilic molecule or can be present both on the amphiphilic molecule as well as in a secondary species that is present within the E-LLC, for example a hydrophobic or hydrophilic monomer or polymer species. The covalent chemical crosslinking of the mesoporous elastomer may also be additionally facilitated by the addition of a crosslinking initiator, such as photo-initiators or a thermal initiator or a mixture of two types.

Physical crosslinking present within the mesoporous elastomer can be achieved via physical chain entanglements between the amphiphilic molecules, or between the amphiphilic molecules and the long polymeric chains of any other polymeric species present within the confined mesoporous domains of the mesoporous elastomer. For example, this can be described as the chain entanglements between the hydrophobic tails of the amphiphilic molecules and any other long-chain polymers that crosslink or polymerize from the hydrophobic monomeric species (Fig. 2). Therefore, the physical crosslinks of the mesoporous elastomer preferably possess at least two of the three types of physical chain entanglements listed below:
(1) Chain entanglements between the amphiphilic molecules themselves. For example, the physical interaction, such as coiling or hydrogen bonding between the hydrophobic tails and/or physical interaction between the hydrophilic heads;
(2) Chain entanglements between the amphiphilic molecules and a secondary polymeric species, such as the physical coiling of the hydrophobic tails of the amphiphilic molecule and long polymeric chains of a crosslinked hydrophobic polymer present within the hydrophobic domains of the mesoporous elastomer and/or the physical coiling of the hydrophilic groups of the amphiphilic molecule and long polymeric chains of a crosslinked hydrophilic polymer present within the hydrophilic domains of the mesoporous elastomer; and
(3) Physical chain entanglements of the secondary polymeric species that are also chemically crosslinked such that the long polymeric chains even though chemically crosslinked at specific points, can also coil and physically interlink with neighboring polymeric chains of the same polymer, that might also be chemically crosslinked.

Physical interactions can be of the type, but not limited to, chain coiling of polymeric species owing to entropy gain and/or the formation of crystalline or semi-crystalline domains of the polymeric regions within the mesoporous structure (Fig. 2 and Fig. 3).

The aforementioned combination of covalent chemical crosslinking and physical chain entanglement crosslinking is important for the mechanical properties of the mesoporous elastomer. Note that the chemical and physical crosslinking present within the mesoporous elastomer is preferably not random. On the contrary, the crosslinks are periodically arranged such that the chemical and physical crosslinks repeat in specific sequence throughout the entire macroscopic volume of the mesoporous elastomer at well-defined nanometer distances (Fig. 3). This periodic arrangement of the chemical and physical crosslinks lends the mesoporous elastomer with an ordered nanostructure and an ordered arrangement of pores.

The effect of the periodic molecular and nano-level structure as well as the combination of the aforementioned (at least two types) of covalent chemical and (at least two types) of physical crosslinks has a distinct effect on the macroscopic mechanics of the mesoporous elastomer. Under tension, the mesoporous elastomer shows two distinct and linear regions on the corresponding stress-strain curve; a low strain-high stiffness region, R1 (strain, < 0.5) and a high strain-low stiffness region, R2 (strain, > 0.5) (Fig. 4). The strain dependent response arises from the cooperative force dissipation enabled by covalent chemical crosslinks and physical chain entanglements taking place in the materials. R1 corresponds to a physical regime where physical chain entanglements dissipate the stresses by viscoelastic deformation. At low strains, the physically entangled chains of the amphiphilic species and the secondary polymeric species slide and stretch thereby absorbing all the stresses incurred by the material. R2 corresponds to a cooperative regime where the force dissipation is via a combinatorial stretching and disruption of the covalent chemical crosslinks and physical chain entanglements, during which elongation reaches its maximum. This is supported by absence of sharp yield point between R1 and R2, which indicates that the physical chain entanglements (or linkages) continue to viscoelastically deform at large deformations in tandem with covalent chemical linkages. The importance of the presence of at least two types of both physical and chemical crosslinks within the mesoporous elastomer is demonstrated by testing the same material with only one type of physical chain entanglements together with only one type of covalent chemical crosslinks (Fig. 4A). Although both materials respond analogously to the applied stress, a mesoporous elastomer with at least two types of the covalent chemical crosslinks and the physical chain entanglements is 10-fold stronger (Stress*ₘₐₓ* = 1.71±0.39 MPa) and 5-fold stiffer (*E_{mod} =* 2.08±1.05 MPa) than the mesoporous elastomer with only one type of physical chain entanglements and only one type of covalent chemical crosslinks (Stress *ₘₐₓ* = 0.26±0.06 MPa and *E_{mod} =* 0.53±0.06 MPa). In addition, the mesoporous elastomer with at least two types of both chemical and physical crosslinks shows 5-times greater elongation, (Strain*ₘₐₓ* ≈ 521 %) than its counterpart (Strain*ₘₐₓ* ≈ 110 %) (Fig. 4A).

The mesoporous elastomer consisting of at least two of the three types of both covalent chemical crosslinks and physical chain entanglement crosslinks can display a strain stiffening effect. Fig. 4B shows that both R1 and R2 enhances for a mesoporous elastomer with the aforementioned description, which implies that the presence of at least two of three types of both physical and covalent crosslinks strengthen the molecular structure of the mesoporous elastomer when the mesoporous elastomer is stretched at different strain rates. For example, at sufficiently large time scales or low strain rates, the physical chain entanglements within the mesoporous elastomer have more time to adapt to forces and deform plastically while shorter time scales (or high strain rates) elicit higher resistance to movement, hence greater stiffness of the physical entanglements. The change can be clearly observed at R1, where the stiffness of the mesoporous elastomer increases by 50% (1.48 MPa -> 3.45 MPa) when the strain rate is increased by a factor of 10 (5 mm min⁻¹ -> 50 mm min⁻¹).

The mesoporous elastomer is also resilient, which can be defined as the ability to recover deformation over multiple tensile or compression cycles. The mesoporous elastomer withstands multiple loadings over deformations exceeding 300% strain without rupture (Fig. 5A). Recovery efficiency (RE) is estimated by obtaining the difference from the loading-unloading hysteresis and the mean recovery for the mesoporous elastomer is 80%. The uniform relaxation from extremely large deformations with a high RE is indicative of the tough molecular network of the mesoporous elastomer that comprises of the covalent chemical and physical chain entanglement crosslinks. RE approaches 100% when the mesoporous elastomer is allowed to relax for a period >5 min (Fig. 5B).

Mesoporous elastomers include a combination of covalent chemical and physical chain entanglement crosslinks and displays a highly elastic and tough mechanical behavior. A third characterizing feature of a mesoporous elastomer is its mesoporosity. A mesoporous elastomer with a chemically and physically crosslinked molecular structure is organized at the nano-mico (meso) structural level as having an ordered arrangement of pores that are monodisperse in size. Mesoporous elastomers can possess mesopores of different pore sizes, typically in the range of a minimum of 2 nm to a maximum of 1000 nm. The pores in a mesoporous elastomer are arranged in a well-ordered fashion and are derived from the E-LLC structure. For example, a mesoporous elastomer can possess pores of the aforementioned sizes in a cylindrical, cubic or lamellar geometry. Note that the pores in a mesoporous elastomer are uniformly present throughout the full macroscopic volume of the mesoporous elastomer and not present only on the surface.

Mesoporous elastomers are also amphiphilic in nature. Owing to the fundamental LLC nature of the mesoporous elastomer, the mesoporous elastomers possess mesopores that are of hydrophilic nature and possess mesopores that are hydrophobic in nature in the same material. These pores are present in a periodic fashion throughout the macroscopic volume of the mesoporous elastomer. Periodic amphiphilic mesopores mean that the mesopores are repeating, such that the pores follow the sequence hydrophilic-hydrophobic-hydrophilic-hydrophobic (Fig. 3).

The aforementioned amphiphilic and mesoporous nature of the mesoporous elastomer enables the chemical or physical binding of substances of hydrophilic, hydrophobic and amphiphilic character within the mesopores of the mesoporous elastomer. Therefore, a mesoporous elastomer with mesoporous structure and with covalent chemical and physical chain entanglement crosslinking can display high mechanical stiffness and elasticity as well as host or bind chemical substances of both hydrophobic and hydrophilic character. These substances can include, but not limited to, organic molecules, such as; medical drugs of antibiotic, anti-inflammatory or anti-cancer nature; organic dyes; nanoparticles of both organic (organic crystals or polymers) and inorganic (ceramics or metals) in nature. The specific substance can be hosted within the mesoporous elastomer at the specific pores depending upon its hydrophilicity or hydrophobicity (Example 8 and Example 9).

In case of the physical binding of organic molecules within the mesopores of the mesoporous elastomer, slow and sustained or controlled release of the same molecule can be achieved from the mesoporous elastomer. In case of physical binding of inorganic nanoparticles, such as ceramics, within the mesopores of the mesoporous elastomer, an increase in mechanical stiffness and handling of the mesoporous elastomer can be achieved. These properties are elaborated in Example 8 and Example 9.

The mesoporous elastomer is substantially non-biodegradable and is substantially non-toxic to human cells. A cell viability of greater than 75% is taken as a measure of non-toxicity of a material toward human cells. The mesoporous elastomer does not deteriorate in mechanical stiffness and elongation on exposing the material to conditions that deviate from ambient temperature and dry conditions. For example, the mesoporous elastomer retains up to 90% of its stiffness and toughness on exposing it to phosphate buffer salt solution at 37 °C for a period of 9 weeks (Example 6).

The mesoporous elastomer can be processed at room temperature (20-25°C) into any desirable shape and form without the necessity for higher temperatures. The term 'processed at room temperature' means that all steps for forming the mesoporous elastomer, which includes, synthesis of the E-LLC precursor, casting or forming the E-LLC precursor and crosslinking of the E-LLC precursor, can be achieved at room temperature. This room temperature processability feature of the material lends the possibility to shape and form the E-LLCs into mesoporous elastomers as macroscopic objects of any complex shape or form, including thin films or bulk 3D objects using techniques, such as 3D printing or injectability or molding or casting processes (Example 7). The aforementioned processes are preferably first performed on the E-LLC followed by crosslinking the material to form the final mesoporous elastomer.

The mesoporous elastomer can also be processed to possess higher level structural features at the microscopic and macroscopic level. For example, via 3D printing, micro sized pores or microscale anisotropy can be incorporated within the mesoporous elastomer. 3D printing can also facilitate the formation of macroscopic objects of defined size and form such as cubes, meshes, grids, thin films, cylinders and other complex geometries (Example 7).

The ordered mesoporous structure of the E-LLC is formed via molecular self-assembly facilitated by hydrophobic interactions between the three species. By applying extrusion shear to the E-LLCs within fine nozzles, the mesoporous structure could be shear-aligned along the printing directions over longer distances as desired. This enabled control of nanostructural anisotropy of the pores over the whole macrostructure of the final mesoporous elastomer such that all pores are aligned along one direction throughout the macroscopic volume of the material.

The effect of mesopore alignment may consequently be reflected on the tensile properties of the mesoporous elastomer where maximum elongation and tensile strength were 100% greater along the direction of orientation compared to the transverse directions (Fig. 9). The results indicated the high level of structural control that could be achieved via 3D printing of the E-LLCs to form hierarchically structured, shape specific and tough mesoporous elastomers. Mesopore alignment may also offer useful traits for the resulting mesoporous elastomer for controlled release of substances hosted within the mesoporous structure.

An embodiment of the mesoporous elastomer is in medical device applications where any 3D form of the mesoporous elastomer can be used as a whole material for a specific device related application, such as mesoporous elastomeric tubes as urinary catheters, locally injectable mesoporous elastomers as orthopedic fillers, form of sheets or 3D fillers of the mesoporous elastomer as a wound dressing, wound fillings or a skin graft. The added property of hosting medically active molecules, such as drugs, proteins or nanoparticles, might also be relevant for the applications in medical devices. For example, the mesoporous elastomers can physically host or encapsulate or chemically bind antibiotics or antimicrobial peptides within its pores for added therapeutic effect of the medical device. Other forms of active substances, such as inorganic nanoparticles or any relevant drug molecule, can also be incorporated within the mesopores of the mesoporous elastomer so as to achieve a specific medically relevant therapeutic or functional effect.

The mesoporous elastomer may be formed in to a device having a tubular form. The mesoporous elastomer may be formed via casting or 3D printing. The tubular form has an inner diameter and an outer diameter. The inner diameter, that is, the diameter of the opening, of the tubular form may be from about 0.1 mm to about 10 mm, such as about 0.5 mm to about 5 mm, such as about 2.5 mm. The outer diameter of the tubular form may be from about 0.2 mm to about 10 mm, such as from about 1 mm to about 10 mm, such as about 5 mm. The tubular element may have a length of greater than 1 mm, such as greater than 10 mm. The tubular form is ideally suited to forming a catheter such as, but not limited to, urinary catheters, intravenous catheters, abdominal catheters, pigtail catheters.

Other embodiments of the mesoporous elastomer might also be observed outside of medical device applications. For example, an application of the mesoporous elastomer is as sealants or joints for cracks whereby the mesoporous elastomer can be applied at a crack and crosslinked to seal of the crack. Furthermore, the mesoporous elastomer can be incorporated with nanoparticles or inorganic ions, such as Ca²⁺ or Fe²⁺, to further release the substances into the cracked region to enhance the sealing of the cracks.

Other embodiments of the mesoporous elastomer is its chemical resistance nature. The mesoporous elastomer, owing to its chemical crosslinks and physical chain entanglement crosslinks, can resist chemical attacks and subsequent dissolution or degradation, for example from harsh acidic, alkali or organic solvents (Table 5).

An antimicrobial agent may be attached to the mesoporous elastomer forming an antimicrobial mesoporous elastomer. The antimicrobial agent may be covalently attached to the mesoporous elastomer. The antimicrobial agent may be covalently attached via carboxyl groups present on the surface of the mesoporous elastomer. The antimicrobial agent may be an amphiphilic molecule having a hydrophilic part and a hydrophobic part. As described the mesoporous elastomer is substantially amphiphilic. The hydrophobic part of the amphiphilic antimicrobial agent may interact with the hydrophobic part of the mesoporous elastomer such that the antimicrobial agent has a non-random orientation. The hydrophilic part of the amphiphilic antimicrobial agent may interact with the hydrophilic part of the mesoporous elastomer. The interaction of both parts of the amphiphilic antimicrobial agent with both parts of the mesoporous elastomer results in the non-random orientation of the antimicrobial agent. The hydrophilic and hydrophobic interactions between the elastomer and the antimicrobial agent also leads to increased stability and improved immobilization of the antimicrobial agent. The antimicrobial agent may be an antimicrobial peptide, such as RRPRPRPRPWWWW-NH₂ (RRP9W4N, Red Glead Discovery AB, Lund, Sweden), RP RRPRPRPRP-NH₂, (RRP9N, Red Glead Discovery AB, Lund, Sweden) RRPRPRPWWWWRP-NH₂ (RRP7W4RPN, Red Glead Discovery AB, Lund, Sweden), or RRPRPWWRPWWRP-NH₂ (RRP5W2RPW2RPN, Red Glead Discovery AB, Lund, Sweden). Sequences for RRP9W4N, RRP9N, are provided in WO 2012/033450 A1. Sequences for RRP7W4RPN (SEQ ID NO:1) and RRP5W2RPW2RPN (SEQ ID NO: 2) are provided together with this application. The antimicrobial peptide may be an antimicrobial peptide comprising less than 20 amino acids which comprises an amino acid sequence having at least 90%, such as 95%, identity to the amino acid sequence RRPRPRPRP (sequence provided in WO 2012/033450 A1), and optionally, a stretch of at least three consecutive tryptophan or phenylalaline residues appended to either the C- or N-terminus, or therebetween. The antimicrobial peptide may comprise an N-terminal amidation. The antimicrobial peptide may be an antimicrobial peptide comprising a stretch of at least one, such as at least three, hydrophobic amino acids, such as phenylaline, or tryptophan residues, forming a hydrophobic region. The hydrophobic region allows for interaction with the hydrophobic regions of the mesoporous elastomer. However, other antimicrobial peptides may be suitable for use as the antimicrobial agent.

The antimicrobial agent may be a synthetically derived AMP as those in the preceding paragraph or biologically derived. Biologically derived AMPs can be derived from a kininogen proteins, proline and arginine rich end leucine rich repeat protein (PRELP), growth factor proteins, coagulation system proteins, complement factor C3a, von Willebrand factor, vitronectin, superoxide dismutase, prion proteins, protein C inhibitor, fibronectin, laminin, chemokines, and histidine rich glycoprotein. Some examples of biologically derived AMPs are human cathelicidin derived LL-37 peptide and Omiganan pentahydrochloride.

As with the mesoporous elastomer without any antimicrobial agent, the antimicrobial mesoporous elastomer may be non-toxic, that is the antimicrobial agent may be non-toxic to human cells.

An antimicrobial mesoporous elastomer may be especially suitable for devices and articles intended for use on or within the human body. For example, the antimicrobial mesoporous elastomer may be especially suitable as an implant, a surgical instrument, a stent, a catheter, a contact lens, hygiene product and as wound care product such as wound dressings or band-aids.

### EXAMPLES

### EXAMPLE 1 - Synthesis of a reverse hexagonal elastomeric lyotropic liquid crystal (E-LLC) with at least two of three crosslinking scenarios both physically and chemically to form the mesoporous elastomer

A reverse hexagonal E-LLC gel was formed by mixing a crosslinkable (or polymerizable) tri-block co-polymer amphiphilic molecule diacrylate modified PLURONIC^{®} P123 (MP123), methyl methacrylate (MMA) and Milli-q water. In this system, MMA is the continuous domain located outside the micellar cylinders while water is located within the hexagonally ordered cylindrical micelles of the polymerizable amphiphile. Small amounts of radical initiators, 2-hydroxy 2-methyl propiophenone (2-4 wt% of MP123)) and benzoyl peroxide (4-6 wt% of MMA), were added to the mixture to facilitate the crosslinking process. The components were mixed thoroughly at a weight ratio of water: MP123 : MMA = 15 : 50 : 35, until a thick homogenous E-LLC gel was formed. Once the E-LLC gel was prepared, it can be either molded or 3D printed into a desired macroscopic shape and size. Following this, the gel was exposed to ultraviolet light (UV, wavelength of 254 nm or 365 nm) for a specific period of time. UV light crosslinks the gel into a tough and elastic mesoporous elastomer material with a highly ordered, hexagonal mesoporous structure, and which can be removed from the mold or surface immediately. The crosslinking process can be carried out even using heat, for example in an oven at 60 degree celsius.

There are at least two and more preferably three different types of chemical crosslinks that form when the E-LLC precursor gel was crosslinked:
(1) Numerous MP123 molecules crosslink with other MP123 molecules within the hexagonal cylindrical micelles;
(2) MMA polymerizes to form PMMA chains which can crosslink with other PMMA chains within the hydrophobic domain; and
(3) MP123 molecules crosslink with PMMA chains at the cylindrical micellar interface.

The chemical crosslinks can be formed using a desired chemistry on the different molecules. In this Example, the crosslinks were formed by radical polymerization of unsaturated carbon-carbon double bonds (C=C) present on the hydrophilic groups of MP123 and on the hydrophobic MMA molecules resulting in the formation of a group of ester bonds (Fig. 2).

In addition to chemical crosslinks, a system of at least two out of three different types of physical crosslinks is also present in the mesoporous elastomer system such as:
(1) Chain entanglement between the different crosslinked PMMA polymeric species within the hydrophobic domains of the mesoporous elastomer;
(2) Chain entanglements between the hydrophobic chains of the MP123; and
(3) Chain entanglements between MP123 molecules and PMMA polymeric chains within the hydrophobic domains of the hexagonal mesoporous elastomer (Fig. 2).

This was seen in FTIR data shown in Fig. 6 and Table 4.

### EXAMPLE 2 - Synthesis of a reverse hexagonal E-LLC with less than two of three crosslinking scenarios both physically and chemically to form a mesoporous elastomer

A reverse hexagonal E-LLC gel was formed by mixing the crosslinkable (or polymerizable) amphiphilic molecule MP123, butyl acetate (BuAc) and Milli-q water. In this system, BuAc was the continuous domain while water was located within the hexagonally ordered cylindrical micelles of the amphiphile. A small amount of radical initiators (2-hydroxy 2-methyl propiophenone 2-4 wt% of MP123 and benzoyl peroxide 4-6 wt% MMA) were added to the mixture to facilitate the crosslinking process. The components were mixed thoroughly at a weight ratio of water :MP123 : BuAc = 15 : 50 : 35, until a thick homogenous gel was formed. Once the E-LLC gel was prepared, it can be either molded or 3D printed into a desired macroscopic shape and size. Following this, the E-LLC gel was exposed to ultraviolet (UV, wavelength of 254 nm or 365 nm) light for a specific period of time. UV light crosslinks the E-LLC gel into a tough and elastic material with a highly ordered, hexagonal mesoporous structure and which can be removed from the mold or surface immediately. The crosslinking process can be carried out even using heat, for example in an oven at 60 °C.

There were less than the required two out of three different types of chemical and physical crosslinks that form when this E-LLC precursor gel was crosslinked. The only chemical crosslink was (1) numerous MP123 molecules crosslink with other MP123 molecules within the micelles via formation of ester bonds by the saturation of -C=C bonds present on the MP123 molecules. The only physical crosslink was (2) chain entanglements between the hydrophobic chains of the MP123.

### EXAMPLE 3 - FITR analysis

Fourier transform infrared spectroscopy (FTIR) of the materials was performed (Perkin Elmer Frontier spectrometer) using the attenuated total reflectance (ATR, GladiATR diamond plate from Pike Technologies) mode over a wavenumber range of 400-4000 cm⁻¹. The samples were 1-2 mm thick and scanned 16 times. FTIR studies further provides strong evidence for the proposed molecular structure of the mesoporous elastomer with at least two types of physical crosslinks and at least two types of chemical crosslinks as elaborated above, where the -C=O bending (749 cm⁻¹) and -O-CH₃ deformation (1435 cm⁻¹) confirmed the presence of PMMA together with the characteristic finger print region of MP123 (841-1373 cm⁻¹) (Table 4). Same results were observed even after thorough washing of the mesoporous elastomer in chloroform to remove unreacted MP123 monomers, MMA monomers as well as any linear chain PMMA polymers that may interfere with the signals. Surprisingly, there was little mass loss (≈15%) after the chloroform wash, which indicated that more than 85% of the molecules were fully crosslinked. Moreover, the dense crosslinking in the mesoporous elastomer made it extremely stable even in harsh alkaline solutions (1M NaOH) for more than 1 week. In contrast, the mesoporous elastomer formed out of the E-LLC gel in Example 2 with its sole MP123-MP123 chemical linkages completely disintegrated within 2 hours (Table 5).

### EXAMPLE 4 - Mesoporous structure of the mesoporous elastomer

Small angle X-ray scattering (SAXS) measurements of both the E-LLC of Example 1 and the E-LLC of Example 2, before and after crosslinking, were performed using a Mat: Nordic SAXS/WAXS/GISAXS instrument at an exposure time of 5 min/sample (SAXSLAB, λ = 1.54 Å) over a q-range of 0.0185-6.81 nm⁻¹. The samples for measurement (gel or crosslinked solid) were loaded into stainless steel sandwich cells sealed with mica sheets.

The E-LLC (Example 1) and the E-LLC (Example 2) showed Bragg peaks corresponding to adjacent peak ratio of 1:3^{1/2}:2:7^{1/2}, typical for LLCs with a hexagonally ordered, micellar mesoporous structure (Fig. 1). Dimensional values of the E-LLC structure, such as fibrillar lattice spacing (Aₕₑₓ), fibril diameter (d_{aq}) and fibril-fibril distance within the hexagon (d_{hyp}) was calculated to be, Aₕₑₓ(Example 1 E-LLC) = 14.16 nm, d_{aq}(Example 1 E-LLC) = 7.90 nm and d_{hyp}(Example 1 E-LLC) = 8.91 nm. The values are approximately close to the dimensional values of the Example 2 E-LLC (Aₕₑₓ(Example 2 E-LLC) = 13.71 nm, d_{aq}(Example 2 E-LLC) = 7.64 nm and d_{hyp}(Example 2 E-LLC) = 8.63 nm). The similarity in the nanostructure and corresponding dimensions confirms that MMA and BuAc behave similarly in the E-LLC phase. Other possible E-LLC mesoporous structures are shown in Table 3, such as micellar cubic or lamellar, formed at different compositions of MP123-water-MMA.

SAXS data of the crosslinked mesoporous elastomer confirmed that the reverse hexagonal nanostructure was preserved after crosslinking (Fig. 1). The mesoporous elastomer showed the [100] peak and less resolved secondary signals. This provides the first indication that, the mesoporous elastomer possesses a new solidifying unit, probably related to the PMMA chains that more intensely causes random X-ray scattering. However, the mesoporous dimensions of the mesoporous elastomer did not significantly change after crosslinking (Aₕₑₓ(Example 1 E-LLC -> Mesoporous elastomer) = 14.16 nm -> 13.64 nm). This indicates that the PMMA and MP123 polymers form and crosslink within the pre-organized nanostructure without disturbing its size.

Other mesoporous structures of the mesoporous elastomer was achieved by mixing the components in the ratio mentioned in Table 3.

### EXAMPLE 5 - Mechanical strength and elasticity of the mesoporous elastomer

Mechanical testing of all materials was performed using an Instron 5600 UTM. All measurements were carried out on a sample size of N≥3. Specimens for the uniaxial tensile testing was prepared by 3D printing the materials into dumbbell shaped samples with measured, length × width, 10 mm × 6 mm. The thickness of all freshly prepared samples was maintained at 1 mm. A slight increase in thickness (≈1.2 mm) was observed for the samples after soaking in aqueous solutions due to the water uptake. The samples were stretched over a range of strain rates (εₜ = 5-50 mm/min) until fracture. The Young's modulus for all samples were calculated at the linear region of the initial strain, between ε = 2-10%. Cyclic tensile measurements with continuous and incremental sweeps were performed at a εₜ of either 2 mm/min or 10 mm/min. The maximum strains measured were 50%, 100%, 150%, 200%, 250%, 300% and 350%. Cyclic tensile tests with intermediate recovery times were performed at a rate of 10 mm/min. Compressive tests for all samples were conducted on cylindrical specimens measuring 4 mm × 4 mm at a strain rate of 0.02 mm/s.

The importance of the presence of at least two types of both physical and chemical crosslinks within the mesoporous elastomer was demonstrated by testing another mesoporous elastomer (as elaborated in Example 2) with only one type of physical chain entanglements and with only one type of covalent chemical crosslinks (Fig. 4A). Although both materials responded analogously to the applied stress, a mesoporous elastomer with at least two types of the covalent chemical crosslinks and at least two types of the physical chain entanglements was 10-fold stronger (σ*ₘₐₓ* = 1.71±0.39 MPa) and 5-fold stiffer (*E_{mod}* = 2.08±1.05 MPa) than the mesoporous elastomer with only one type of physical chain entanglements and only one type of covalent chemical crosslinks (σ*ₘₐₓ* = 0.26±0.06 MPa and *E_{mod} =* 0.53±0.06 MPa). In addition, the mesoporous elastomer with at least two types of both chemical and physical crosslinks showed 5-times greater elongation, (ε*ₘₐₓ* ≈ 521 %) than its counterpart (ε*ₘₐₓ* ≈ 110 %) (Fig. 4A and Fig. 4B).

The mesoporous elastomer withstood multiple loadings over deformations exceeding 300% strain without rupture (Fig. 5). Recovery efficiency (RE) was estimated by obtaining the difference from the loading-unloading hysteresis and the mean recovery for the mesoporous elastomer was 80%. The uniform relaxation from extremely large deformations with a high RE is indicative of the tough molecular network of the mesoporous elastomer that comprised the covalent chemical and physical chain entanglement crosslinks. RE approached 100% when the mesoporous elastomer was allowed to relax for a period >5 min (Fig. 5).

### EXAMPLE 6: Biodegradation and mechanical stability of the mesoporous elastomer

Biodegradation tests were performed on the mesoporous elastomer samples by placing a few pieces of the material in 20 mM phosphate buffered saline (PBS, pH= 7.00, 37 degree celsius) for a period of 10 weeks. The PBS media was replaced at regular intervals of 1 week. The degradation was quantified by measuring the weight loss of the mesoporous elastomer over time. Both wet weight and dry weight was measured in order to determine the exact % mass loss. Samples were dried at room temperature (RT, 20-25 degree Celsius) for 24 h before measuring the dry weight. To evaluate the change in tensile properties against the biodegradation, tensile test specimens as per aforementioned dimensions were prepared. Each specimen was stretched to a point (ε = 300%) within the failure limit at a strain rate of 5 mm/min. Following this, the specimens were relaxed and soaked in 20 mM PBS for 1, 2 and 9 weeks. The soaked samples were removed at each time point and immediately re-stretched unto the failure limit.

The results showed that the Young's modulus of the mesoporous elastomer was unaffected up to the measured period of 9 weeks (Fig. 7A-B). However, a noticeable reduction in maximum elongation was observed at 9 weeks (492%->310%), which indicated slow breakdown of covalent ester bonds in the mesoporous elastomer. A gradual mass loss in the elastomer by 15-18% over a period of 10 weeks was observed. The degradation was a result of the water uptake capacity (W_{H2O}) of the mesoporous elastomer, where hydrophilic mesoporous domains enabled water diffusion (W_{H2O} = 35.12%) into the mesoporous structure, which when combined with salts and higher temperatures catalyzed the breakage of ester bonds. A linear extrapolation of the degradation curve indicated an average half-life (time for 50% mass loss) of 30-40 weeks for the mesoporous elastomer (Fig. 7C).

### EXAMPLE 7: Room temperature processing: 3D printing and injectability of the reverse hexagonal E-LLC gels and crosslinking to form mesoporous elastomers of specific shape and form

E-LLC gels in the syringe were 3D printed using an INKREDIBLE 3D Bioprinter (CELLINK) into desired shapes onto a glass substrate using 400 µm graded nozzle. Specific shapes and geometries were designed using custom-written G-codes, which are available on request. The E-LLC gels were crosslinked for 20-30 minutes using UV (wavelength of 252 nm, 90 W) or by placing the gel in a sealed enclosure at 60 degree Celsius for 24 h.

The E-LLC gels were extrusion-3D printed (e-3DP) to produce macroscopic and shape-specific mesoporous elastomers. Fig. 8 shows 3D printed Mesoporous elastomers with high degree of geometrical complexity, such as 3D grids, hollow cylinder and a scaled down, anatomical model of a human nose. The objects were printed using an extrusion 3D printer driven by a pneumatic air valve through 400 µm nozzles. The porous mesh structure shown in the Fig. 8 demonstrated the fine structural control achieved at the sub mm scale. The printed mesoporous elastomers had a rubbery texture and was tough and elastic under continuous deformations, such as twisting and compression forces (Fig. 8). Compressive modulus (*E_{c}mod*) and ultimate compressive strength (σ _{c}) of the 3D printed samples was as high as 12.09±3.21 MPa and 86.66±4.22 MPa, respectively. Moreover, compressive properties of UV crosslinked (RT for 20 min) and heat crosslinked (60 °C for 24 h) mesoporous elastomers did not differ greatly and confirmed that UV exposure for 20 min was sufficient for complete crosslinking and high strength exhibited by the mesoporous elastomer.

In addition to the shape and geometric control at macroscale, the shear response of the E-LLC enabled remarkable control of the nanostructural anisotropy. As mentioned above, the ordered mesoporous structure of the E-LLC was formed via molecular self-assembly facilitated by hydrophobic interactions between MP123, water and MMA. By applying extrusion shear to the E-LLCs within fine nozzles, the mesoporous structure could be shear-aligned along the printing directions over longer distances as desired. This enabled control of nanostructural anisotropy of the pores over the whole macrostructure of the final mesoporous elastomer such that all pores are aligned along one direction throughout the macroscopic volume of the material. Fig. 9 validated this concept using 2D scanning SAXS data of a 1 mm × 1 mm region from the 3D printed cylinder shown in Fig. 8 marked with a square. The SAXS analysis was performed over 48 points on the printed sample to analyze the anisotropy of the mesopores over mm length scales. Each data point (2D spectra) was averaged and converted to an image representing the direction and degree of orientation along the macroscopic printed filaments. The direction and degree of pore orientation perfectly matched the macroscale printed direction, while the fundamental hexagonal geometry of the mesoporous structure remained undisturbed (Fig. 9). The effect of mesopore alignment was consequently reflected on the tensile properties of the mesoporous elastomer where maximum elongation and tensile strength were 100% greater along the direction of orientation (σ *ₘₐₓ* = 1.71±0.39 MPa, ε*ₘₐₓ*= 425.25±87.44 %) compared to the transverse directions (σ *ₘₐₓ* = 0.62±0.05 MPa, ε*ₘₐₓ* = 170.26±26.47 %) (Fig. 9). The results indicated the high level of structural control that could be achieved via 3D printing of the E-LLCs to form hierarchically structured, shape specific and tough mesoporous elastomers.

One can draw parallels between the structure of the mesoporous elastomer to biological soft tissues like muscle and tendons, where fibrous structures are ordered from the nano to the macroscale, eventually displaying directional properties. Furthermore, the room temperature processing of the mesoporous elastomers extended their use as injectable materials. A proof-of-concept was demonstrated where the E-LLC was injected into a cylindrical defect within a dead chicken flesh. On UV-crosslinking using biologically relevant wavelengths (λ = 364 nm) for 10-15 min, the E-LLC became turbid and transformed into a tough solid filling with good physical adhesion to the surrounding flesh. The method can potentially be extended to any type of tissue defects and can be 3D printed directly into a defect with tailored structural features, such as specific mesopore orientation and micro-porosity that might support better functionalities.

### EXAMPLE 8: Physical binding and sustained release of hydrophilic and hydrophobic drugs from the pores of the mesoporous elastomer

A hydrophobic anti-inflammatory drug, Ibuprofen, was loaded into the hydrophobic pores of the mesoporous elastomer by soaking the mesoporous elastomer in a solution of 100 mg/ml solution of lbupofen in acetone. The mesoporous elastomer was soaked for 24 h for the Ibuprofen to be loaded into the mesoporous elastomer. After 24 h, the mesoporous elastomer with Ibuprofen/acetone, was dried at room temperature to remove the acetone. Following acetone removal, the mesoporous elastomer was placed in a quartz cuvette containing sodium dodecyl sulphate (SDS) solution with gentle shaking. At specific time point, the cuvette containing the mesoporous elastomer/lbuprofen in SDS was exposed to light in UV/Visible spectrophotometer (λ = 200-400 nm). The absorption was measured and the amount of Iburprofen released from the mesoporous elastomer was monitored over time. As seen from Fig. 10, the Ibuprofen followed a sustained release from the mesoporous elastomer over a period of 15-20 days.

A hydrophilic antibiotic drug, Vancomycin, was loaded into the hydrophilic pores of the mesoporous elastomer by soaking the mesoporous elastomer in a solution of 100 mg/ml solution of Vancomycin in Milli-q water. The mesoporous elastomer was soaked for 24 h for the Vancomycin to be loaded into the mesoporous elastomer. Following loading, the mesoporous elastomer was placed in quartz cuvette containing pure Milli-q water with gentle shaking. At specific time point, the cuvette containing the mesoporous elastomer/Vancomycin in water was exposed to light in UV/Visible spectrophotometer (λ = 200-400 nm). The absorption measured the amount of Vancomycin released from the mesoporous elastomer over time. As seen from Fig. 11, the Vancomycin followed a sustained release from the mesoporous elastomer over a period a period of 1-2 days. The difference between Vancomycin release and Ibuprofen release can be attributed to the hydrophilicity of the Vancomycin, which contributed to the faster diffusion of the drug from the mesopores of the mesoporous elastomer, when released in an aqueous environment.

As described above the mesoporous elastomer and/or the antimicrobial mesoporous elastomer, may be a carrier for a therapeutic agent. The carrier may be used as a medicament.

### EXAMPLE 9 - Physical binding of inorganic hydroxyapatite nanoparticle, within the pores of the mesoporous elastomer

Hydroxyapatite nanoparticles were incorporated into the hydrophilic mesopores of the mesoporous elastomer for improving the mechanical and functional properties of the mesoporous elastomer (Fig. 12A). The hydroxyapatite was incorporated into the mesoporous elastomer by first preparing a modified version of the hexagonal E-LLC gel, using the same protocol as described above in Example 1. The main difference was that Milli-q water was replaced with 2.1 M Ca²⁺ (Ca/P = 1.67) solution during the preparation of base E-LLC gel. Final composition of the modified E-LLC gel was MP123 : 2.1 M Ca²⁺ solution : MMA at 50% : 15% : 35% by weight. The modified E-LLC gel was then cast, molded or 3D printed to obtain the desired shape followed by UV crosslinking for 20 min to form the mesoporous elastomer. Next, the mesoporous elastomer was exposed to NH₃ gas at a pressure of 2 bar (99.999%, Hi-Q 5.0, AGA Gas) in a sealed autoclave for 3h. The final materials showed a pale-yellow appearance indicative of calcium phosphate or hydroxyapatite formation.

On XRD analysis, the mesoporous elastomer with hydroxyapatite showed broad Bragg peaks corresponding to nanocrystals of hydroxyapatite (HAp) (Fig. 12B). The material contained ≈7wt% HAp, a relatively small amount owing to the low amounts of aqueous phase (≈15 wt% calcium phosphate precursor solution) used to prepare the precursor E-LLC gel. In spite of the low HAp content, the mesoporous elastomer with HAp was 2-fold stiffer compared to its mesoporous elastomer without HAp. The increase in stiffness can be attributed to the ordered manner, in which the HAp nanocrystals reinforced the polymeric mesoporous structure of the mesoporous elastomer. The HAp particles and the chemically and physically crosslinked polymeric chains created a uniform inorganic-organic interface at the mesoscale, which translated into alternating soft-hard regions (Fig. 12A). Remarkably, the increase in stiffness did not compromise the toughness (or maximum elongation), which steadily kept at ≥300% (Fig. 12C). The periodicity of the organic-inorganic regions of the mesoporous elastomer with HAp enabled complete elastic recovery within an hour of elongation to 250% strain (Fig. 12D). Thus, a mesoporous structure of the mesoporous elastomer offered great flexibility for manipulating the composition of the mesoporous elastomer to achieve a desired function.

### EXAMPLE 10 - Evaluating cytotoxicity of the mesoporous elastomer

A cryopreserved vial containing HDFa (human dermal fibroblast) cells was removed from a liquid nitrogen storage and placed on ice. The vial was then thawed in a 37°C water bath until the ice was melted. The cells were then pipetted into a 25 cm² cell culturing flask containing Medium 106, 10% fetal bovine serum (FBS), 1 µg/mL hydrocortisone, 10 ng/mL human epidermal growth factor, 3 ng/mL basic fibroblast growth factor, 10 µg/mL heparin, 10 µg/mL gentamicin and 0.25 µg/mL amphotericin B. They were then stored in 37°C and 5 % CO₂. When needed, the medium was discarded and replaced with 5 mL of the same solution. This needs to be done due to that the cells use up the nutrients from the growth medium and also discards waste to it.

When the cells reached a confluency of around 80-90% in the flask they were subcultured. This was done by first discarding the media where the cells were present. 2 mL Dulbecco's phosphate-buffered saline (DPBS) was then added for around 10 seconds and then removed again. This was used to rinse the cell culture from chelators. 4 mL of Trypsin/EDTA (0.025% trypsin and 0.01% EDTA in PBS) was then added for around 20 seconds. 3 mL of the solution was then removed and the cells were incubated at room temperature together with the remaining 1 mL of Trypsin/EDTA. This was done for around 5 minutes, until the cells started to round up and detach from the surface. The flask was gently shaken in order for the cells to detach completely. 1 mL of growth medium was then added to stop the trypsinization. The solution was then transferred to a falcon tube and was centrifuged for 5 minutes at 200 RCF. The supernatant was removed and the cells were resuspended in 1 mL of growth medium. A Bürker chamber was used to calculate the number of cells in the solution and around 75 000 cells were then transferred into a new 25 cm² culturing flask containing 5 mL growth medium. The flask was then again incubated at 37°C and 5 % CO₂ and when needed the medium was discarded and replaced with 5 mL of the same solution.

Small pieces of the mesoporous elastomer were exposed to the growth media of fibroblast cells. The samples were kept in the growth media for at least one day in order to potentially release any toxic compounds from the material into the growth media. The falcon tubes were occasionally vortexed in order to make this process more effective.

An MTT assay was performed on the cells grown in the exposed growth media and compared against a control media. The MTT assay was started by culturing cells in a 96-well plate together with 0.2 mL sample-exposed growth media. The growth media that was used was Dulbecco Modified Eagle Medium (DMEM) without phenol red and with addition of 1 v/v% 200mM L-Glutamine, 10 v/v% FBS, 1 µg/mL hydrocortisone, 10 ng/mL human epidermal growth factor, 3 ng/mL basic fibroblast growth factor, 10 µg/mL heparin, 10 µg/mL gentamicin and 0.25 µg/mL amphotericin B. The reason why Medium 106 wasn't used was due to that it contained phenol red which could affect the results. A positive control was also included, containing cells together with growth media which had not been in contact with any sample. The cells were cultured inside a humidified chamber, 37°C and 5 % CO₂, until they reached a cell count of around 30,000-50,000 cells.

The growth media was then discarded from each well and replaced with 100 µL of the same media that was used previously. 10 µL 12 mM MTT solution was then added to each well. A negative control was also included, containing 10 µL MTT solution together with 100 µL of pure growth media. The plate was then incubated at 37°C and 5 % CO₂ for 4 hours. 100 µL SDS-HCl solution was then added to each well and all wells were mixed thoroughly by using the pipette. The SDS-HCl solution was made by mixing 1 gram of SDS with 10 mL 0.01 M HCl. The plate was then again incubated at 37°C and 5 % CO₂ for 4 hours. Each well was then mixed again with a pipette and the absorbance at 570 nm was measured using a microplate reader.

It was observed that the cell viability of cells exposed to mesoporous elastomer soaked growth media was on average greater than 75%. This was indicative of the non-toxicity and biocompatibility of the mesoporous elastomer.

### EXAMPLE 11 - Differential scanning calorimetry of the mesoporous elastomer

Differential scanning calorimetry (DSC, Mettler Toledo) was performed on the samples in alumina crucibles and heating rate of 10 K min⁻¹ from - 80 °C to 170 °C (N₂) for two cycles. Thermal analysis of the Mesoporous Elastomer formed out the E-LLC of Example 1 showed ≤ 7 wt% water content specifying the almost dry state in its as-synthesized form. Differential scanning calorimetry (DSC) of the mesoporous elastomer shows a broad endothermic rise in heat flow between -70°C to +120 °C, without sharp transition points. This implies the predominant amorphous character of the material. In contrast, the mesoporous elastomer formed out of E-LLC of Example 2 shows sharp melting and glass transitions corresponding to the semi-crystalline character of the sole MP123-MP123 chemical crosslinks (Fig. 16). Both materials display a very low, initial glass transition at, -63.41 °C (T_{g2} for the mesoporous elastomer from E-LLC of Example 1) and -65.24 °C (T_{g1} for the mesoporous elastomer from E-LLC of Example 2). This transition corresponds to the chain movements (T_{g}) of the PEO-PPO-PEO blocks of the MP123 networks. Following this, the mesoporous elastomer of Example 1 deviates from that of Example 2 and shows a gradual increase in heat flow up to 150 °C, indicative of the highly amorphous character of the PEO-PPO groups and the presence of an interphase element that prevents it from crystallizing. The small peak at 24.89 °C (T*) for the mesoporous elastomer of Example 1 is presumably the melting point of crystalline polyethylene oxide (PEO) domains, which can be clearly observed in the mesoporous elastomer of Example 2 (Tₘ₁ = 28.73 °C). However, the broad latent heat and gradual endothermic increase up to 150 °C proves the much lower crystallinity of PEO domains in the mesoporous elastomer of Example 1 is due to interphase crosslinking of MP123-PMMA chains.

### Example 12 - Synthesis of a reverse hexagonal E-LLC and formation of a mesoporous elastomer

A reverse hexagonal E-LLC gel was formed by mixing the crosslinkable (or polymerizable) tri-block co-polymer amphiphilic molecule MP123, styrene and Milli-q water. In this system, styrene was the continuous domain located outside the micellar cylinders while water was located within the hexagonally ordered cylindrical micelles of the polymerizable amphiphile. Small amounts of radical initiators (2-hydroxy 2-methyl propiophenone (2-4 wt% of MP123) and benzoyl peroxide (4-6 wt% of Styrene)) were added to the mixture to facilitate the crosslinking process. The components were mixed thoroughly at a weight ratio of water : MP123 : styrene = 15 : 50 : 35, until a thick homogenous gel was formed. Once the gel was prepared, it can be either molded or 3D printed into a desired macroscopic shape and size. Following this, the gel was exposed to ultraviolet light (UV, wavelength of 254 nm or 365 nm) for a specific period of time. UV light crosslinked the gel into a tough and elastic mesoporous elastomer material with a highly ordered, reverse hexagonal mesoporous structure, and which could be removed from the mold or surface immediately. The crosslinking process can be carried out even using heat, for example in an oven at 60 °C. There were three different types of chemical crosslinks that formed when the E-LLC precursor gel was crosslinked. (1) Numerous MP123 molecules crosslinked with other MP123 molecules within the hexagonal cylindrical micelles. (2) Styrene polymerized to form polystyrene chains, which could crosslink with other polystyrene chains within the hydrophobic domain. (3) MP123 molecules crosslinked with polystyrene chains at the cylindrical micellar interface. The chemical crosslinks were formed using a desired chemistry on the different molecules. In this specific example, the crosslinks were formed by radical polymerization of unsaturated carbon-carbon double bonds (C=C) present on the hydrophilic groups of MP123 and on the hydrophobic styrene molecules resulting in the formation of a group of ester bonds. In addition to chemical crosslinks, a system of three different types of physical crosslinks was also present in the mesoporous elastomer system. (1) chain entanglement between the different crosslinked polystyrene polymeric species within the hydrophobic domains of the mesoporous elastomer. (2) Chain entanglements between the hydrophobic chains of the MP123. (3) Chain entanglements between MP123 molecules and polystyrene polymeric chains within the hydrophobic domains of the hexagonal mesoporous elastomer.

### Example 13: Synthesis of a normal hexagonal E-LLC and the formation of a mesoporous elastomer

A normal hexagonal E-LLC gel was formed by mixing the crosslinkable (or polymerizable) tri-block co-polymer amphiphilic molecule MF127, MMA and Milli-q water. In this system, Milli-q water was the continuous domain located outside the micellar cylinders, while MMA was located within the hexagonally ordered cylindrical micelles of the polymerizable amphiphile. Small amounts of radical initiators (2-hydroxy 2-methyl propiophenone (2-4 wt% of MF127) and benzoyl peroxide (4-6 wt% MMA)) were added to the mixture to facilitate the crosslinking process. The components were mixed thoroughly at a weight ratio of water : MF127 : MMA = 50 : 35 : 15, until a thick homogenous gel was formed. Once the gel was prepared, it can be either molded or 3D printed into a desired macroscopic shape and size. Following this, the gel was exposed to ultraviolet light (UV, wavelength of 254 nm or 365 nm) for a specific period of time. UV light crosslinked the gel into a tough and elastic mesoporous elastomer material with a highly ordered, normal hexagonal mesoporous structure, and which could be removed from the mold or surface immediately. The crosslinking process can be carried out even using heat, for example in an oven at 60 °C. There were three different types of chemical crosslinks that formed when the E-LLC precursor gel was crosslinked. (1) Numerous MF127 molecules crosslinked with other MF127 molecules outside the hexagonal cylindrical micelles. (2) MMA polymerized to form PMMA chains, which could crosslink with other PMMA chains within the hydrophobic domain (inside the cylinders). (3) MF127 molecules crosslinked with PMMA chains at the cylindrical micellar interface. In this specific example, the crosslinks were formed by radical polymerization of unsaturated carbon-carbon double bonds (C=C) present on the hydrophilic groups of MF127 and on the hydrophobic MMA molecules resulting in the formation of a group of ester bonds. In addition to chemical crosslinks, a system of three different types of physical crosslinks was also present in the mesoporous elastomer system. (1) Chain entanglement between the different crosslinked PMMA polymeric species within the hydrophobic domains of the mesoporous elastomer. (2) Chain entanglements between the hydrophobic chains of the MF127. (3) Chain entanglements between MF127 molecules and PMMA polymeric chains within the hydrophobic domains of the hexagonal mesoporous elastomer.

**Table 3 - Compositions of E-LLC used to prepare mesoporous elastomers with different mesoporous geometries**

| | **Nanostructure** | **MP123 (wt%)** | **Water (wt%)** | **BuAc (wt%)** | **MMA (wt%)** |
|---|---|---|---|---|---|
| E-LLC (Example 2) | Hexagonal mesoporous | 50 | 15 | 35 | - |
| | Lamellar 1 (L_{α1}) | 30 | 60 | 10 | - |
| | Lamellar 2 (L_{α2}) | 50 | 30 | 20 | - |
| | Micellar cubic (I₂) | 35 | 12.5 | 52.5 | - |
| E-LLC (Example 1) | Hexagonal mesoporous | 50 | 15 | - | 35 |
| | Lamellar 1 (L_{α1}) | 30 | 60 | - | 10 |
| | Lamellar 2 (L_{α2}) | 50 | 30 | - | 20 |
| | Micellar cubic (I₂) | 35 | 12.5 | - | 52.5 |

**Table 4 - Correlation of FTIR signal to the chemical vibration in the different samples**

| Wavenumber (cm⁻¹) | Pure PMMA | Pure MP123 | Mesoporous elastomer (washed in CHCl₃) |
|---|---|---|---|
| 482 | In plane bending C-C-O | | Present |
| 749 | C=O out of plane bending | | Present |
| 808 | C-O sym stretch | | Present |
| 841 | CH₂ rocking | CH₂ rocking | Present |
| 991 | C-O stretch and in plane bending of OCH₃ | | Present |
| 1016 & 1061 | | C-O-C asym stretch and CH₂ sym. rock | Present |
| 1104 | | C-O-C sym stretch | Present |
| 1145 | asym Stretch C-O-C | asym stretch C-O-C C-C stretch | Present |
| 1192 | | | |
| 1345 | | CH₂ asym wag | Present |
| 1373 | | CH₃ sym deformation | Present |
| 1387 | In plane sym def of aCH₃ | | Weak signal |
| 1435 | In plane sym def of OCH₃ | | Presence of all signals especially matching PMMA at 1435 and 1485 and DAP123 and 1449-1452 |
| 1450 | In plane asym defn of OCH₃ | CH₂ scissor | |
| 1485 | In plane asym def of aCH₃ | | |
| 1722 | C=O stretch | C=O stretch | Present |
| 2868 | C-H sym stretch in CH₂ | C-H sym stretch in CH₂ | Present |
| 2950 | C-H asym stretch in CH₂ | C-H asym stretch in CH₂ | Present |
| 2971 | | C-H stretch in CH₃ | Present |
| 2997 | | C-H asym stretch in aCH₃ | Present, weak signal |

**Table 5 - Stability of mesoporous elastomer in chloroform and 1M sodium hydroxide.**

| | **Initial weight (mg)** | **Final weight (mg)** | **Loss (%)** |
|---|---|---|---|
| Mesoporous elastomer (Example 1) in CHCl₃, 2 days | 57.2 | 48.6 | 15.0 |
| Mesoporous elastomer (Example 1) in 1M NaOH, 1 week | 8.9 | 7.8 | 12.4 |
| Mesoporous elastomer (Example 2) in 1M NaOH, 1 week | Completely disintegrated within 3 hours | | |

### Example 14: UV Crosslinking Methods and Parameters to crosslink the Mesoporous Elastomer

**Table 6. Experimental variable table shows parameters varied to study the injectability and UV crosslinking of the Mesoporous Elastomer. Note that the wt% of initiator refers to the respective amounts of 2 hydroxy 2 methyl propiophenone and benzoyl peroxide.**

| | |
|---|---|
| Photoinitiator concentration, wt% with respect to MP123 and MMA | 4, 2, 1, 0.4, 0.04 |
| UV exposure time, min | 2, 4, 6, 8, 10, 12, 15, 20, 25 |
| Distance from the UV source, cm | 0, 1, 2, 3, 4, 5, 6, 7, 8 |

The E-LLC precursor gel was prepared as described in Example 1 and 2 and was manually extruded onto a glass slide, or injected into a cylindrical mould, depending on the desired defect model. For all experiments a Teflon mould with cylindrical holes measuring 12 × 5 mm or 5 × 5 mm was used. Prior to loading the gels into the cylindrical mould, glass slides were taped to the bottom of the Teflon sheets leaving only the top part of the gels to exposure. This model was chosen to mimic the defect systems observed in biological tissues where crosslinking happens only from one direction. To bring the photopolymerization experimental conditions closer to the prospective processing method (3D printing) all experiments were performed by using the UV LED curing system with biologically relevant wavelength (UVA, λ=365 nm) installed on a 3D bioprinter (INKREDIBLE, CELLINK, Sweden).

For all of the following experiments, the variation of the distance between the UV light source and the sample i.e UV intensity was achieved by placing the Teflon sheet onto the printing bed, and adjusting the distance between the sample surface and the UV light source to the desired value (Table 6). The distance between the lamp and sample was correlated to the intensity of the UV light using the data provided by the manufacturer (Fig 19).

The Mesoporous Elastomer samples were subjected to compression testing on an Instron 5600 UTM universal testing machine. Samples were prepared into 5 × 5 mm cylinders and compressed to 70 % strain at a rate of 1 mm/min, or until the limit of the load cell (100 N) was exceeded. The respective stress-strain diagrams were recorded, and the E-modulus was calculated at the linear regions of the curves. In all cases, the data below strain = 5 % were excluded due to non-uniform behaviour of the material at the surface, probably caused by surface inhomogeneities. To correlate the crosslinking parameters to the degree of crosslinking, the Mesoporous Elastomer samples were subjected to leaching experiments in water medium. Prior to immersing the samples in water, they were left to air dry in room temperature in a fume hood to remove any excess solvents. The initial dry weight was recorded. Afterwards each sample was immersed in a vial of 10 ml Milli-Q water and continuously shaken using a mechanical shaker at room temperature for 7, 24 and 72 h, respectively. After each soaking period, the samples were air dried at 37 °C, and repeatedly immersed into a fresh water medium. Sample weight was recorded after each soaking period and each drying period, ultimately calculating the weight loss with respect to the initial dry weight. All experiments were performed using triplicates.

**Table 7. Effect of Light Intensity and Initiator Conc. on Crosslinking - Quantitative analysis through Compression Testing**

| Distance from the UV source, cm | Initiator concentration | | |
|---|---|---|---|
| | 0.4 wt% | | 0.04 wt% |
| | E-modulus 1, MPa | E-modulus 2, MPa | E-modulus, MPa |
| 5 | 2.30 | | 0.27 |
| 6 | 2.63 | 2.50 | 0.29 |
| 7 | 3.47 | 3.43 | 0.26 |
| 8 | 3.70 | 3.18 | 0.23 |

**Table 8. Effect of UV exposure time on Crosslinking - Quantitative analysis through Compression Testing: Initiator conc. 0.4 wt% and distance (Light Intensity) at a distance of 6 cm from UV source.**

| Time of UV exposure, min | E-modulus 1, MPa | E-modulus 2, MPa |
|---|---|---|
| 6 | 0.26 ± 0.09 | |
| 8 | 1.28 ± 0.18 | |
| 10 | 2.63 ± 0.12 | 2.50 ± 0.23 |
| 15 | 8.24 ± 1.13 | 4.11 ± 0.27 |
| 20 | 10.39 ± 0.43 | 5.19 ± 0.26 |

Further results are shown in Figures 18-20.

### Summary of results

The mechanical properties of the Mesoporous Elastomer can be tailored by controlling the crosslinking parameters of the precursor E-LLC material. Three parameters can have a significant effect on the crosslinking of the mesoporous elastomer, namely UV exposure time, UV light intensity and photo initiator concentration.

A minimum photoinitiator content of 0.01 wt% with respect to the MP123 and MMA concentration and maximum photoinitator content of 10 wt% with respect to the MP123 and MMA can be used to form the mesoporous elastomer within a compressive stiffness range of 0.01-100 MPa.

The mesoporous elastomer can be crosslinked via UV exposure within a suitable time range of 5 min - 20 min. More preferably, the UV exposure time for the crosslinking of the mesoporous elastomer is a minimum of 5 seconds to a maximum of 30 min.

The mesoporous elastomer can be crosslinked using different intensities of the UV light such that a suitable range of UV intensity might lie between 5mW/cm² to 45mW/cm².

The mesoporous elastomer is crosslinked to different degrees of crosslinking such that a low initiator concentration (0.01-1 wt%), long UV exposure time (1-10 min) and low intensities (5-20 mW/cm²) yield materials of high stiffnesses such as in the range of 1-100 MPa whereas a high initiator concentration (1-10 wt%), short UV exposure times (1-5 min) and high intensities greater than 40mW/cm² might yield materials of lower stiffnesses such as in the range of 0.1-2 MPa.

Depending upon the UV parameter and crosslinking method, the crosslink density can be estimated. A low crosslinking density possesses residual content that can be lost when exposed to liquids (10-50% loss in mass), whereas a high crosslinked sample exhibits high stability and only negligible losses upon exposure to liquids (0-10% loss in mass).

### Example 15 - Covalent attachment of Antimicrobial peptide (AMP) to Mesoporous Elastomer and corresponding antimicrobial activity

An E-LLC material of reverse hexagonal (H₂) lyotropic liquid crystal structure was formed using MP123, methyl methacrylate (MMA) and water as described in Examples 1 and 2.

Two different photoinitiators were used to initiate the photopolymerization. 2-hydroxy 2-methyl propiophenone (2H2MPP) was added to the MP123 to facilitate the polymerization of diacryated Pluronic^{™}, whilst benzoyl peroxide (BP), (Fischer Scientific) was chosen to facilitate the MMA polymerization. Before the E-LLC gel precursor preparation, MMA stock solution was prepared by dissolving a predetermined amount of BP according to the desired weight percentage. Based on previous optimization trials concentration of both photoinitiators was set to 0.04 wt% and kept constant throughout all experiments.

The E-LLC precursor gel was prepared by manually mixing MP123/MMA/water according to the set ratio described in Example 1. The mixing resulted in a thick homogenous gel. The gel was subsequently spread onto a glass slide and sandwiched together with a second glass slide by using a thin spacer. Prepared slides were stored overnight to facilitate the amphiphile self-assembly into a reversed hexagonal structure. Afterwards the prepared E-LLC gels were photo cross-linked for 20 min by using an UV LED light source (UVA, λ=365 nm, 9 W) mounted on a 3D bioprinter (INKREDIBLE, CELLINK, Sweden) forming solid, opaque and homogeneous mesoporous elastomer sheets. Circular sample disks were prepared from the sheets by using a biopsy punch (Ø 8 mm). Finally, the disks were washed in Milli-Q for 48 h to remove any residual fractions like unreacted monomer, photoinitiator or loosely cross-linked polymer chains.

### Example 16 - Surface treatment for antimicrobial activity

In order to analyse the AMP interaction with the mesoporous elastomer, 6 different sample types were introduced: 1) control samples, 2) plasma treated control samples, 3) covalently attached AMP samples, 4) plasma treated and covalently attached AMP samples, 5) physically loaded AMP samples and 6) plasma treated and physically loaded AMP samples. Each sample type was prepared, treated and analysed in a triplicate as shown in below Table.

**Table. Sample types, treatments and the analysis methods carried out.**

| **Sample type** | **Oxygen plasma treatment** | **EDC/NHS activation** | **AMPs** | **UV-Vis analysis** | **LIVE/DEAD staining** |
|---|---|---|---|---|---|
| **Control** | x | - | - | x | x |
| | - | - | - | x | x |
| **Covalently attached AMPs** | x | x | x | x | x |
| | - | x | x | x | x |
| **Physically loaded AMPs** | x | - | x | x | - |
| | - | - | x | x | - |

### Surface plasma treatment

Based on the chemical functionalization potential of the mesoporous elastomer's surface due to the carboxyl group presence, it was hypothesized that the surface activity, the covalent AMP attachment and subsequent antimicrobial activity could be enhanced by introducing additional surface carboxyl groups. This hypothesis was analysed by introducing a prior treatment step of the mesoporous elastomer surface with high energy oxygen plasma, in order to increase the amount of active surface carboxyl groups.

Plasma treatment was carried out with Expanded Plasma Cleaner by Harrick Plasma, USA. Previously washed mesoporous elastomer sample disks were removed from the washing liquid, blotted with a tissue and placed in the Plasma Cleaner chamber onto glass slides. The chamber was evacuated, and oxygen gas was released. The gas flow was controlled by a Plasmaflo accessory attached to the cleaner and the pressure monitored by a digital pressure meter. To ensure plasma ignition pressure was reduced to approx. 200 mTorr. RF-plasma power was set to high (corresponding to power of 30/45 W) and treatment time set to 10 sec.

After the treatment, each sample disk was placed in a separate well of a sterile 24 well plate. Depending on the sample type, disks were either: 1) covered by 1 ml Milli-Q water or 2) subjected to carboxyl group activation by EDC/NHS sequence. These steps were performed immediately after sample treatment to minimize the surface relaxation, contamination and reduction of hydrophilicity.

### Surface carboxyl group activation and Covalent attachment of AMPs to Mesoporous Elastomer

To render the mesoporous elastomer's surface with active carboxyl groups for direct AMP attachment, carbodiimide conjugation chemistry was used.

The mesoporous elastomer disks were removed from the washing solutions, blotted and placed in separate wells of sterile 24 well plates. 1-Ethyl-3-(3-dimethylaminopropyl) carbodiimide (EDC) and N-hydroxysuccinimide (NHS) solutions were prepared by dissolving the required amount of the reagent (target concentration of 2 mg/ml) in 2-(N-morpholino)ethanesulfonic acid (MES) buffer with a pH of 6. Immediately after mixing, 500 µl of EDC solution and 500 µl of NHS solution were pipetted on top of the sample disks. Samples were activated for 30 min under continuous shaking on a shaker plate. Afterwards, activation solutions were thoroughly removed from the disks and each sample washed with 1 ml pure MES buffer twice.

The AMP used was a 13 amino acid peptide of the sequence RRPRPRPRPWWWW-NH₂. A solution of the AMP was prepared by dissolving the required amount of AMP powder (target concentration of 200 µM) in Milli-Q water. Ultimately 500 µl of the AMP solution was pipetted on top of the sample disks followed by activation for 2 h under continuous shaking on a shaker plate. After the activation period AMP soaking solution was aspirated and samples were washed twice with 1 ml and once with 500 µl Milli-Q water collecting the washing water.

### Peptide quantification by UV-Vis spectroscopy

To quantify the AMP take-up by the mesoporous elastomer based on the treatment type, UV-Vis spectroscopy studies were carried out. After AMP attachment, the AMP soaking solutions as well as the washing water was thoroughly collected from all sample types, forming 3 ml of total sample volume. UV-Vis measurements were performed in quartz cuvettes with Thermo Fischer spectrophotometer with the absorbance registered at λ=280 nm (absorption region of tryptophan). To correlate the registered absorbance values to actual AMP concentrations, an external standard curve was prepared and used for all calculations. Additionally, the originally prepared AMP stock solution was analysed to calculate the actual AMP take-up of the samples.

### Bacteria cultivation

The bacterial strains that was used to assess the antimicrobial activity of AMP treated mesoporous elastomers was *Staphylococcus Aureus* CCUG 10778. *S*. *aureus* was chosen due to its high prevalence and pathogenicity in variety of biofilm associated infections (including catheter associated urinary tract infections, CAUTIs). All bacteria were stored frozen at -80 °C.

Colony cultivation was done by streaking Brain-Heart Infusion (BHI) agar plates under sterile technique and incubating them upside down at 37 °C overnight. For all further tests one bacteria colony was inoculated in 5 ml of tryptic soy broth media (TSB, 3% w/v) and incubated at 37 °C to reach bacteria log phase (OD ≈ 0.55 - 0.7) with approx. 10⁹ colony forming units/ml. Afterwards small volume of bacteria solution was sampled and diluted to 10⁶ CFU/ml in fresh TSB media by suspending and vortexing. 1 ml of the bacteria dilution was added to each well containing sample disks and incubated static for 24 h in 37 °C in order to favour biofilm formation. After incubation period the bacteria solution was carefully aspirated, and samples washed twice with 1 ml phosphate-buffered saline (PBS) to remove any planktonic bacteria and avoid disrupting the formed biofilm.

### LIVE/DEAD staining

To analyse the antimicrobial activity of mesoporous elastomer's surface LIVE/DEAD staining method was used to quantify the portion of live and dead bacteria. This method is based on the permeability of live and dead cell membranes to certain fluorescent dyes. LIVE/DEAD BacLight Bacterial Viability Kit L7007 (Thermo Fischer) was used throughout all experiments. This kit consisted of two types of dyes preformulated in two components of certain dye concentrations ("LlVE/DEAD^{®} BacLight Bacterial Viability Kits L7007," 2004). SYTO^{®} 9 is a green-fluorescent nucleic acid stain capable of penetrating both dead and alive cells, whilst propidium iodide is a red-fluorescent nucleic acid stain capable of penetrating only dead cell membranes. A staining solution consisting of 2.5 µl of component A and 0.5 µl of component B was prepared in 1 ml of 0.85 % NaCl aqueous solution. 8 µl of the staining solution was pipetted on top of the washed sample disks collected after the incubation and covered with glass coverslips for fluorescent imaging.

Disks were imaged by using ZEISS Axio fluorescence microscope at wavelengths 500-550 nm and 570-640 nm for live and dead cells, respectively. A large population of images was collected to give a good overall representation of the sample surface. Ultimately, the images were analysed by a macro in imaged software developed by the Centre for Cellular Imaging (CCI), Gothenburg, Sweden that gave quantitative results of the ratio of dead and alive bacteria surface coverage.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality.

## Claims

1. A mesoporous elastomer having an ordered and periodic nanoporous structure, the mesoporous elastomer being formed by a crosslinked elastomeric lyotropic liquid crystal, the elastomeric lyotropic liquid crystal formed by: polymerizable amphiphilic molecules, a hydrophilic solvent, a hydrophobic solvent, wherein the hydrophilic solvent comprises hydrophilic monomers and/or the hydrophobic solvent comprises hydrophobic monomers, and wherein the mesoporous elastomer comprises each of the three chemical covalent crosslinking species:
(1) homogenous chemical crosslinking of polymerizable amphiphilic molecules with neighboring polymerizable amphiphilic molecules;
(2) interfacial and heterogeneous chemical crosslinking of polymerizable amphiphilic molecules with hydrophobic monomers that further form hydrophobic polymer chains and/or interfacial and heterogeneous chemical crosslinking of polymerizable amphiphilic molecules with hydrophilic monomers that further form hydrophilic polymer chains; and
(3) homogenous chemical crosslinking of hydrophobic monomers to form polymeric crosslinked hydrophobic polymer chains and/or homogenous chemical crosslinking of hydrophilic monomers to form polymeric crosslinked hydrophilic polymer chains.

2. The mesoporous elastomer according to claim 1, wherein the mesoporous elastomer possesses a combination of covalent chemical crosslinks and physical chain entanglement crosslinks.

3. The mesoporous elastomer according to claim 1 or 2, wherein the mesoporous elastomer is formed via the molecular self-assembly and intermolecular crosslinking of polymerizable amphiphilic molecules and the mixture of hydrophilic and hydrophobic solvents.

4. The mesoporous elastomer according to any of the claims 1 to 3, wherein the mesoporous elastomer comprises polymerizable amphiphilic molecules that
(1) possess one or more hydrophilic parts as well as one or more hydrophobic parts in the same molecule;
(2) possess a polymerizable group on the hydrophilic part and/or the hydrophobic part; and
(3) self-assemble into lyotropic liquid crystal micellar architectures, such as cubic, hexagonal or lamellar structures, in the presence of a mixture of a hydrophilic solvent and a hydrophobic solvent.

5. The mesoporous elastomer according to any of the claims 1 to 4, wherein the mesoporous elastomer comprises ordered nanoporous structure having mesoscale anisotropy enabling formation of articles possessing direction dependent mechanical properties.

6. The mesoporous elastomer according to any of the claims 1 to 5, wherein the mesoporous elastomer is substantially non-biodegradable.

7. The mesoporous elastomer according to any of the claims 1 to 6, obtainable according to the process of:
mixing polymerizable amphiphilic molecules, a hydrophilic solvent and a hydrophobic solvent to form an elastomeric lyotropic liquid crystal, preferably in the form of a non-viscous liquid like dispersion or viscous gel having an ordered and periodic mesoporous structure throughout its entire macroscopic volume, wherein the hydrophilic solvent comprises hydrophilic monomers and/or the hydrophobic solvent comprises hydrophobic monomers; and
crosslinking the elastomeric lyotropic liquid crystal, preferably using light, heat, crosslinking catalyst and/or a crosslinking or polymerization initiator, to form the mesoporous elastomer.

8. The mesoporous elastomer according to any of the claims 1 to 7, wherein the mesoporous elastomer comprises at least one, preferably at least two and more preferably all, of the three physical chain entanglement species:
(1) homogenous physical chain entanglements of hydrophobic parts of polymerizable amphiphilic molecules and/ or homogenous physical chain entanglements of hydrophilic parts of polymerizable amphiphilic molecules;
(2) homogenous chain entanglements of hydrophobic polymeric chains and/or homogenous chain entanglements of hydrophilic polymeric chains; and
(3) heterogeneous physical chain entanglements between hydrophobic polymeric chains and hydrophobic parts of polymerizable amphiphilic molecules and/or heterogeneous physical chain entanglements between hydrophilic polymeric chains and hydrophilic parts of polymerizable amphiphilic molecules.

9. The mesoporous elastomer according to any of the claims 1 to 8, wherein the mesoporous elastomer has an ordered nanoporous structure having mesoscale, microscale and milliscale anisotropy.

10. The mesoporous elastomer according to any of the claims 1 to 9, wherein the mesoporous elastomer is substantially non-toxic.

11. A method of producing a mesoporous elastomer according to any of claims 1 to 10, wherein the method comprises:
mixing polymerizable amphiphilic molecules, a hydrophilic solvent and a hydrophobic solvent to form an elastomeric lyotropic liquid crystal, preferably in the form of a non-viscous liquid like dispersion or viscous gel having an ordered and periodic mesoporous structure throughout its entire macroscopic volume, wherein the hydrophilic solvent comprises hydrophilic monomers and/or the hydrophobic solvent comprises hydrophobic monomers; and,
crosslinking the elastomeric lyotropic liquid crystal, preferably using light, heat, crosslinking catalysts and/or a crosslinking or polymerization initiator, to form the mesoporous elastomer.

12. An elastomeric lyotropic liquid crystal, wherein the elastomeric lyotropic liquid crystal is a non-viscous liquid like dispersion or viscous gel having an ordered and periodic mesoporous structure throughout its entire macroscopic volume, wherein the elastomeric lyotropic liquid crystal is formed by a mixture of polymerizable amphiphilic molecules, a hydrophilic solvent and a hydrophobic solvent, wherein the hydrophilic solvent comprises hydrophilic monomers and/or the hydrophobic solvent comprises hydrophobic monomers.

13. A method of producing an elastomeric lyotropic liquid crystal according to claims 12, wherein the method comprises mixing polymerizable amphiphilic molecules, a hydrophilic solvent and a hydrophobic solvent to form the elastomeric lyotropic liquid crystal, preferably in the form of a non-viscous liquid like dispersion or viscous gel having an ordered and periodic mesoporous structure throughout its entire macroscopic volume, wherein the hydrophilic solvent comprises hydrophilic monomers and/or the hydrophobic solvent comprises hydrophobic monomers.

14. A method of producing an article comprising a mesoporous elastomer according to any of claims 1 to 10, wherein the method comprises:
casting, molding and/or applying extrusion shear to an elastomeric lyotropic liquid crystal according to claim 12; and
crosslinking the elastomeric lyotropic liquid crystal to form the article comprising or made at least partly of the mesoporous elastomer, having a nanostructural anisotropy, preferably such that pores are aligned along one direction throughout the macroscopic volume of the mesoporous elastomer.

15. The method according to claim 14, wherein the method comprises:
3D printing or injecting, preferably applying extrusion shear, to an elastomeric lyotropic liquid crystal according to claim 12 to form a 3D printed or injected elastomeric lyotropic liquid crystal; and
crosslinking the 3D printed or injected elastomeric lyotropic liquid crystal to form the article comprising or made at least partly of the mesoporous elastomer.

## Patentansprüche

1. Mesoporöses Elastomer, das eine geordnete und periodische nanoporöse Struktur aufweist, wobei das mesoporöse Elastomer durch einen vernetzten elastomeren lyotropen Flüssigkristall gebildet wird, wobei der elastomere lyotrope Flüssigkristall gebildet wird durch: polymerisierbare amphiphile Moleküle, ein hydrophiles Lösungsmittel, ein hydrophobes Lösungsmittel, wobei das hydrophile Lösungsmittel hydrophile Monomere umfasst und/oder das hydrophobe Lösungsmittel hydrophobe Monomere umfasst, und wobei das mesoporöse Elastomer jede der drei chemischen kovalenten Vernetzungsarten umfasst:
(1) homogene chemische Vernetzung von polymerisierbaren amphiphilen Molekülen mit benachbarten polymerisierbaren amphiphilen Molekülen;
(2) grenzflächige und heterogene chemische Vernetzung von polymerisierbaren amphiphilen Molekülen mit hydrophoben Monomeren, die weiter hydrophobe Polymerketten bilden, und/oder grenzflächige und heterogene chemische Vernetzung von polymerisierbaren amphiphilen Molekülen mit hydrophilen Monomeren, die weiter hydrophile Polymerketten bilden; und
(3) homogene chemische Vernetzung von hydrophoben Monomeren zur Bildung von polymeren vernetzten hydrophoben Polymerketten und/oder homogene chemische Vernetzung von hydrophilen Monomeren zur Bildung von polymeren vernetzten hydrophilen Polymerketten.

2. Mesoporöses Elastomer nach Anspruch 1, wobei das mesoporöse Elastomer eine Kombination aus kovalenten chemischen Vernetzungen und physikalischen Kettenverschlingungsvernetzungen aufweist.

3. Mesoporöses Elastomer nach Anspruch 1 oder 2, wobei das mesoporöse Elastomer durch die molekulare Selbstanordnung und intermolekulare Vernetzung von polymerisierbaren amphiphilen Molekülen und der Mischung aus hydrophilen und hydrophoben Lösungsmitteln gebildet wird.

4. Mesoporöses Elastomer nach einem der Ansprüche 1 bis 3, wobei das mesoporöse Elastomer polymerisierbare amphiphile Moleküle umfasst, die
(1) einen oder mehrere hydrophile Teile sowie einen oder mehrere hydrophobe Teile in demselben Molekül besitzen;
(2) eine polymerisierbare Gruppe auf dem hydrophilen Teil und/oder dem hydrophoben Teil besitzen; und
(3) sich in Gegenwart einer Mischung aus einem hydrophilen Lösungsmittel und einem hydrophoben Lösungsmittel selbst zu lyotropen Flüssigkristallmizellenarchitekturen, wie beispielsweise kubischen, hexagonalen oder lamellaren Strukturen, selbst anordnen.

5. Mesoporöses Elastomer nach einem der Ansprüche 1 bis 4, wobei das mesoporöse Elastomer eine geordnete nanoporöse Struktur mit mesoskaliger Anisotropie aufweist, die die Bildung von Gegenständen mit richtungsabhängigen mechanischen Eigenschaften ermöglicht.

6. Mesoporöses Elastomer nach einem der Ansprüche 1 bis 5, wobei das mesoporöse Elastomer im Wesentlichen nicht biologisch abbaubar ist.

7. Mesoporöses Elastomer nach einem der Ansprüche 1 bis 6, erhältlich gemäß dem folgenden Verfahren:
Mischen von polymerisierbaren amphiphilen Molekülen, einem hydrophilen Lösungsmittel und einem hydrophoben Lösungsmittel, um einen elastomeren lyotropen Flüssigkristall zu bilden, vorzugsweise in Form einer nicht-viskosen Flüssigkeit wie einer Dispersion oder eines viskosen Gels, der eine geordnete und periodische mesoporöse Struktur in seinem gesamten makroskopischen Volumen aufweist, wobei das hydrophile Lösungsmittel hydrophile Monomere umfasst und/oder das hydrophobe Lösungsmittel hydrophobe Monomere umfasst; und
Vernetzen des elastomeren lyotropen Flüssigkristalls, vorzugsweise unter Verwendung von Licht, Wärme, Vernetzungskatalysator und/oder einem Vernetzungs- oder Polymerisationsinitiator, um das mesoporöse Elastomer zu bilden.

8. Mesoporöses Elastomer nach einem der Ansprüche 1 bis 7, wobei das mesoporöse Elastomer mindestens eine, vorzugsweise mindestens zwei und noch bevorzugter alle drei physikalischen Kettenverschlingungsarten umfasst:
(1) homogene physikalische Kettenverschlingungen von hydrophoben Teilen von polymerisierbaren amphiphilen Molekülen und/oder homogene physikalische Kettenverschlingungen von hydrophilen Teilen von polymerisierbaren amphiphilen Molekülen;
(2) homogene Kettenverschlingungen aus hydrophoben Polymerketten und/oder homogene Kettenverschlingungen aus hydrophilen Polymerketten; und
(3) heterogene physikalische Kettenverschlingungen zwischen hydrophoben Polymerketten und hydrophoben Teilen von polymerisierbaren amphiphilen Molekülen und/oder heterogene physikalische Kettenverschlingungen zwischen hydrophilen Polymerketten und hydrophilen Teilen von polymerisierbaren amphiphilen Molekülen.

9. Mesoporöses Elastomer nach einem der Ansprüche 1 bis 8, wobei das mesoporöse Elastomer eine geordnete nanoporöse Struktur aufweist, die eine mesoskalige, mikroskalige und milliskalige Anisotropie aufweist.

10. Mesoporöses Elastomer nach einem der Ansprüche 1 bis 9, wobei das mesoporöse Elastomer im Wesentlichen ungiftig ist.

11. Verfahren zur Herstellung eines mesoporösen Elastomers nach einem der Ansprüche 1 bis 10, wobei das Verfahren folgendes umfasst:
Mischen von polymerisierbaren amphiphilen Molekülen, einem hydrophilen Lösungsmittel und einem hydrophoben Lösungsmittel, um einen elastomeren lyotropen Flüssigkristall zu bilden, vorzugsweise in Form einer nicht-viskosen Flüssigkeit wie einer Dispersion oder eines viskosen Gels, der eine geordnete und periodische mesoporöse Struktur in seinem gesamten makroskopischen Volumen aufweist, wobei das hydrophile Lösungsmittel hydrophile Monomere umfasst und/oder das hydrophobe Lösungsmittel hydrophobe Monomere umfasst; und,
Vernetzen des elastomeren lyotropen Flüssigkristalls, vorzugsweise unter Verwendung von Licht, Wärme, Vernetzungskatalysatoren und/oder einem Vernetzungs- oder Polymerisationsinitiator, um das mesoporöse Elastomer zu bilden.

12. Elastomerer lyotroper Flüssigkristall, wobei der elastomere lyotrope Flüssigkristall eine nicht-viskose Flüssigkeit wie eine Dispersion oder ein viskoses Gel ist, die bzw. das eine geordnete und periodische mesoporöse Struktur in ihrem bzw. seinem gesamten makroskopischen Volumen aufweist, wobei der elastomere lyotrope Flüssigkristall durch eine Mischung aus polymerisierbaren amphiphilen Molekülen, einem hydrophilen Lösungsmittel und einem hydrophoben Lösungsmittel gebildet wird, wobei das hydrophile Lösungsmittel hydrophile Monomere umfasst und/oder das hydrophobe Lösungsmittel hydrophobe Monomere umfasst.

13. Verfahren zur Herstellung eines elastomeren lyotropen Flüssigkristalls nach Anspruch 12, wobei das Verfahren das Mischen polymerisierbarer amphiphiler Moleküle, eines hydrophilen Lösungsmittels und eines hydrophoben Lösungsmittels umfasst, um den elastomeren lyotropen Flüssigkristall zu bilden, vorzugsweise in Form einer nicht-viskosen flüssigkeitsartigen Dispersion oder eines viskosen Gels, das eine geordnete und periodische mesoporöse Struktur in seinem gesamten makroskopischen Volumen aufweist, wobei das hydrophile Lösungsmittel hydrophile Monomere umfasst und/oder das hydrophobe Lösungsmittel hydrophobe Monomere umfasst.

14. Verfahren zur Herstellung eines Gegenstands, umfassend ein mesoporöses Elastomer nach einem der Ansprüche 1 bis 10, wobei das Verfahren folgendes umfasst:
Gießen, Formen und/oder Anwenden von Extrusionsscherkraft auf einen elastomeren lyotropen Flüssigkristall nach Anspruch 12; und
Vernetzen des elastomeren lyotropen Flüssigkristalls, um den Gegenstand zu bilden, der das mesoporöse Elastomer umfasst oder mindestens teilweise daraus hergestellt ist und eine nanostrukturelle Anisotropie aufweist, vorzugsweise so, dass die Poren entlang einer Richtung im gesamten makroskopischen Volumen des mesoporösen Elastomers ausgerichtet sind.

15. Verfahren nach Anspruch 14, wobei das Verfahren Folgendes umfasst:
3D-Drucken oder Injizieren, vorzugsweise Anwenden einer Extrusionsscherkraft, auf einen elastomeren lyotropen Flüssigkristall nach Anspruch 12, um einen 3D-gedruckten oder injizierten elastomeren lyotropen Flüssigkristall zu bilden; und
Vernetzen des 3D-gedruckten oder injizierten elastomeren lyotropen Flüssigkristalls, um den Gegenstand zu bilden, der das mesoporöse Elastomer umfasst oder zumindest teilweise daraus besteht.

## Revendications

1. Élastomère mésoporeux présentant une structure nanoporeuse ordonnée et périodique, l'élastomère mésoporeux étant formé par un cristal liquide lyotrope élastomère réticulé, le cristal liquide lyotrope élastomère étant formé par : des molécules amphiphiles polymérisables, un solvant hydrophile, un solvant hydrophobe, dans lequel le solvant hydrophile comprend des monomères hydrophiles et/ou le solvant hydrophobe comprend des monomères hydrophobes, et dans lequel l'élastomère mésoporeux comprend chacune des trois espèces chimiques de réticulation covalente :
(1) réticulation chimique homogène de molécules amphiphiles polymérisables avec des molécules amphiphiles polymérisables voisines ;
(2) réticulation chimique interfaciale et hétérogène de molécules amphiphiles polymérisables avec des monomères hydrophobes qui forment en outre des chaînes de polymères hydrophobes et/ou réticulation chimique interfaciale et hétérogène de molécules amphiphiles polymérisables avec des monomères hydrophiles qui forment en outre des chaînes de polymères hydrophiles ; et
(3) réticulation chimique homogène de monomères hydrophobes pour former des chaînes de polymères hydrophobes réticulés polymères et/ou réticulation chimique homogène de monomères hydrophiles pour former des chaînes de polymères hydrophiles réticulés polymères.

2. Élastomère mésoporeux selon la revendication 1, dans lequel l'élastomère mésoporeux possède une combinaison de réticulations chimiques covalentes et de réticulations d'enchevêtrement de chaîne physique.

3. Élastomère mésoporeux selon la revendication 1 ou 2, dans lequel l'élastomère mésoporeux est formé via l'auto-assemblage moléculaire et la réticulation intermoléculaire de molécules amphiphiles polymérisables et le mélange de solvants hydrophiles et hydrophobes.

4. Élastomère mésoporeux selon l'une quelconque des revendications 1 à 3, dans lequel l'élastomère mésoporeux comprend des molécules amphiphiles polymérisables qui
(1) possèdent une ou plusieurs parties hydrophiles ainsi qu'une ou plusieurs parties hydrophobes dans la même molécule ;
(2) possèdent un groupe polymérisable sur la partie hydrophile et/ou la partie hydrophobe ; et
(3) s'auto-assemblent en architectures micellaires à cristaux liquides lyotropes, telles que des structures cubiques, hexagonales ou lamellaires, en présence d'un mélange d'un solvant hydrophile et d'un solvant hydrophobe.

5. Élastomère mésoporeux selon l'une quelconque des revendications 1 à 4, dans lequel l'élastomère mésoporeux comprend une structure nanoporeuse ordonnée présentant une anisotropie à mésoéchelle permettant la formation d'articles possédant des propriétés mécaniques dépendant de la direction.

6. Élastomère mésoporeux selon l'une quelconque des revendications 1 à 5, dans lequel l'élastomère mésoporeux est sensiblement non biodégradable.

7. Élastomère mésoporeux selon l'une quelconque des revendications 1 à 6, pouvant être obtenu selon le processus de :
mélange des molécules amphiphiles polymérisables, d'un solvant hydrophile et d'un solvant hydrophobe pour former un cristal liquide lyotrope élastomère, de préférence sous la forme d'une dispersion de type liquide non visqueux ou d'un gel visqueux présentant une structure mésoporeuse ordonnée et périodique dans tout son volume macroscopique, dans lequel le solvant hydrophile comprend des monomères hydrophiles et/ou le solvant hydrophobe comprend des monomères hydrophobes ; et
réticulation du cristal liquide lyotrope élastomère, de préférence en utilisant de la lumière, de la chaleur, un catalyseur de réticulation et/ou un initiateur de réticulation ou de polymérisation, pour former l'élastomère mésoporeux.

8. Élastomère mésoporeux selon l'une quelconque des revendications 1 à 7, dans lequel l'élastomère mésoporeux comprend au moins une, de préférence au moins deux et plus préférentiellement la totalité, des trois espèces d'enchevêtrement de chaîne physique :
(1) enchevêtrements de chaînes physiques homogènes de parties hydrophobes de molécules amphiphiles polymérisables et/ou enchevêtrements de chaînes physiques homogènes de parties hydrophiles de molécules amphiphiles polymérisables ;
(2) enchevêtrements de chaînes homogènes de chaînes de polymères hydrophobes et/ou enchevêtrements de chaînes homogènes de chaînes de polymères hydrophiles ; et
(3) enchevêtrements de chaînes physiques hétérogènes entre les chaînes de polymères hydrophobes et les parties hydrophobes de molécules amphiphiles polymérisables et/ou enchevêtrements de chaînes physiques hétérogènes entre les chaînes de polymères hydrophiles et les parties hydrophiles de molécules amphiphiles polymérisables.

9. Élastomère mésoporeux selon l'une quelconque des revendications 1 à 8, dans lequel l'élastomère mésoporeux présente une structure nanoporeuse ordonnée présentant une anisotropie à mésoéchelle, à échelle micrométrique et à échelle millimétrique.

10. Élastomère mésoporeux selon l'une quelconque des revendications 1 à 9, dans lequel l'élastomère mésoporeux est sensiblement non toxique.

11. Procédé de production d'un élastomère mésoporeux selon l'une quelconque des revendications 1 à 10, dans lequel le procédé comprend :
le mélange de molécules amphiphiles polymérisables, d'un solvant hydrophile et d'un solvant hydrophobe pour former un cristal liquide lyotrope élastomère, de préférence sous la forme d'une dispersion de type liquide non visqueux ou d'un gel visqueux présentant une structure mésoporeuse ordonnée et périodique dans tout son volume macroscopique, dans lequel le solvant hydrophile comprend des monomères hydrophiles et/ou le solvant hydrophobe comprend des monomères hydrophobes ; et,
la réticulation du cristal liquide lyotrope élastomère, de préférence en utilisant de la lumière, de la chaleur, des catalyseurs de réticulation et/ou un initiateur de réticulation ou de polymérisation, pour former l'élastomère mésoporeux.

12. Cristal liquide lyotrope élastomère, dans lequel le cristal liquide lyotrope élastomère est une dispersion de type liquide non visqueux ou un gel visqueux présentant une structure mésoporeuse ordonnée et périodique dans tout son volume macroscopique, dans lequel le cristal liquide lyotrope élastomère est formé par un mélange de molécules amphiphiles polymérisables, d'un solvant hydrophile et d'un solvant hydrophobe, dans lequel le solvant hydrophile comprend des monomères hydrophiles et/ou le solvant hydrophobe comprend des monomères hydrophobes.

13. Procédé de production d'un cristal liquide lyotrope élastomère selon la revendication 12, dans lequel le procédé comprend le mélange de molécules amphiphiles polymérisables, d'un solvant hydrophile et d'un solvant hydrophobe pour former le cristal liquide lyotrope élastomère, de préférence sous la forme d'une dispersion de type liquide non visqueux ou d'un gel visqueux présentant une structure mésoporeuse ordonnée et périodique dans tout son volume macroscopique, dans lequel le solvant hydrophile comprend des monomères hydrophiles et/ou le solvant hydrophobe comprend des monomères hydrophobes.

14. Procédé de production d'un article comprenant un élastomère mésoporeux selon l'une quelconque des revendications 1 à 10, dans lequel le procédé comprend :
le coulage, le moulage et/ou l'application d'un cisaillement par extrusion à un cristal liquide lyotrope élastomère selon la revendication 12 ; et
la réticulation du cristal liquide lyotrope élastomère pour former l'article comprenant ou composé, au moins en partie, de l'élastomère mésoporeux, présentant une anisotropie nanostructurale, de préférence telle que les pores sont alignés le long d'une direction dans tout le volume macroscopique de l'élastomère mésoporeux.

15. Procédé selon la revendication 14, dans lequel le procédé comprend :
l'impression 3D ou l'injection, de préférence l'application d'un cisaillement par extrusion, à un cristal liquide lyotrope élastomère selon la revendication 12 pour former un cristal liquide lyotrope élastomère imprimé en 3D ou injecté ; et
la réticulation du cristal liquide lyotrope élastomère imprimé en 3D ou injecté pour former l'article comprenant ou composé, au moins en partie, de l'élastomère mésoporeux.
